# EUROPEAN PATENT APPLICATION

(11) **EP 2 601 945 A1**
(43) Date of publication of application: **12.06.2013**
(21) Application number: 11191921.3
(22) Date of filing: 05.12.2011
(51) Int. Cl.: A61K 31/138, A61P 27/16, A61P 39/00, G01N 33/15

(54) **Inhibition of cis-platin induced ototoxicity**

(71) Applicant: Thomale, Jürgen, 45138 Essen (DE); Hanenberg, Helmut, Indianapolis, IN 46278 (US)
(72) Inventor: Thomale, Jürgen, 45138 Essen (DE); Mendus, Diana, Palo Alto, CA 94301 (US)
(74) Representative: Elbel, Michaela

(57) **Abstract**

The invention describes diphenylhydramine (DPH) for treating ototoxicity in a subject, for inhibiting the formation of platinum adducts in a cell of the inner ear, and for preventing and/or inhibiting structural and functional damage in the inner ear. Furthermore, the invention describes an *in vitro* method for determining a subject's susceptibility for platinum induced ototoxicity and/or platinum induced nephrotoxicity and an *in vitro* method for screening a compound for inhibiting ototoxicity induced by a platinum complex as well as a kit useful for screening. The invention also describes a composition for treating cancer and concurrently inhibiting ototoxicity, nephrotoxicity and/or neurotoxicity. The invention further describes DPH for treating nephrotoxicity in a subject induced by a medication comprising a platinum complex.

## Description

### Field of the invention

The invention relates to diphenylhydramine (DPH) for treating ototoxicity in a subject, for inhibiting the formation of platinum adducts in a cell of the inner ear, and for preventing and/or inhibiting structural and functional damage in the inner ear. Furthermore, the invention relates to an agent for treating ototoxicity induced by a platinum complex and to an *in vitro* method for screening a compound for inhibiting ototoxicity induced by a platinum complex as well as to a kit useful for screening. The invention also relates to a composition for treating cancer and concurrently inhibiting ototoxicity, nephrotoxicity and/or neurotoxicity. The invention further relates to DPH for treating nephrotoxicity in a subject induced by a medication comprising a platinum complex.

### Background of the invention

Novel compounds and medications as well as novel applications of known compounds are continuously developed to provide therapies and possibly cures to human diseases. Especially in medical fields concerning complex diseases with poor prognosis increasingly efficient medications are needed. Cancer drugs, for example, need to eliminate as many cancer cells as possible in a single medical application, since surviving cells may repopulate due to uncontrolled proliferation, leading to a reoccurrence of the cancer. The most efficient medications are, however, particularly aggressive and also damage or even kill cells of normal tissue, further impairing the patient's health condition. In general, two approaches are used to cope with these problems. The first is to avoid systemic distribution of the medication by limiting the effect of the medication to the specific location of the disease, such that in the best case only the tissue affected by the disease is targeted by the medication. This approach, however, is only possible where a disease is restricted to a specific area of the patient's body and the medication can be applied locally. Since this is often not the case, a second approach focuses on specifically inhibiting the side effect(s) of a given medication. Amongst the side effects often observed with aggressive medications, especially with these used for cancer treatment, comprise neurotoxicity, nephrotoxicity and ototoxicity. Ototoxicity and nephrotoxicity are observed to occur frequently for certain cancer treatments but are also known to sporadically occur upon the administration of other medications, as e.g. antibiotics and diuretics. In any case, both are considered particularly severe side effects, because they cause permanent damages like deafness and renal insufficiency. Thus, there is a strong need of therapeutic approaches and means to inhibit such side effects.

### Summary of the invention

In a first aspect, the invention is directed to diphenylhydramine (DPH) for treating ototoxicity in a subject, wherein the ototoxicity is induced by a medication.

In a second aspect, the invention is directed to diphenylhydramine (DPH) for inhibiting the formation of platinum adducts in a cell of the inner ear.

In a further aspect, the invention is directed to diphenylhydramine (DPH) for preventing and/or inhibiting structural and functional damage in the inner ear during a platinum-based treatment.

In a further aspect, the invention is directed to an agent for treating ototoxicity induced by a platinum complex, wherein the agent interferes with a modulator of the import and/or export of the platinum complex into/from a cell.

In a further aspect, the invention is directed to diphenylhydramine (DPH) for treating nephrotoxicity in a subject induced by a medication comprising a platinum complex, wherein DPH inhibits the formation of platinum adducts within a cell of the kidney, preferably of the kidney cortex, more preferred a proximal tubular cell.

In a further aspect, the invention is directed to diphenylhydramine (DPH) for treating ototoxicity and/or nephrotoxicity in a subject, wherein the ototoxicity and/or nephrotoxicity is induced by a medication comprising a platinum complex.

In a further aspect, the invention is directed to an *in vitro* method for determining a subject's susceptibility for platinum induced ototoxicity and/or platinum induced nephrotoxicity, comprising the steps of determining the subject's genetic variant of a platinum complex transporter, and correlating the genetic variant of the platinum complex transporter to the susceptibility for platinum induced ototoxicity and/or platinum induced nephrotoxicity.

In a further aspect, the invention is directed to an *in vitro* method for screening a compound capable of inhibiting ototoxicity, nephrotoxicity and/or neurotoxicity induced by a platinum complex, comprising the steps of providing a test sample comprising cells derived from the organ of Corti, the stria vascularis of the inner ear and/or the proximal tubules of the kidney, incubating the test sample with the compound, adding the platinum complex to the test sample, determining the amount of platinum adducts formed by the platinum complex in the test sample and comparing the level of platinum adducts formed in the test sample to the level of platinum adducts formed in a control sample, wherein a reduced amount of platinum adducts formed in the test sample in comparison to the control sample indicates the compound's potential to inhibit ototoxicity induced by a platinum complex.

In a further aspect, the invention is directed to a kit for screening a compound, comprising cells derived from the organ of Corti, the stria vascularis of the inner ear and/or the proximal tubules of the kidney, a platinum complex, and an antibody against platinum adducts.

In a further aspect, the invention is directed to a composition of treating cancer and concurrently inhibiting ototoxicity, nephrotoxicity and/or neurotoxicity, wherein the composition comprises cis-platin and diphenylhydramine (DPH).

### Brief description of the drawing

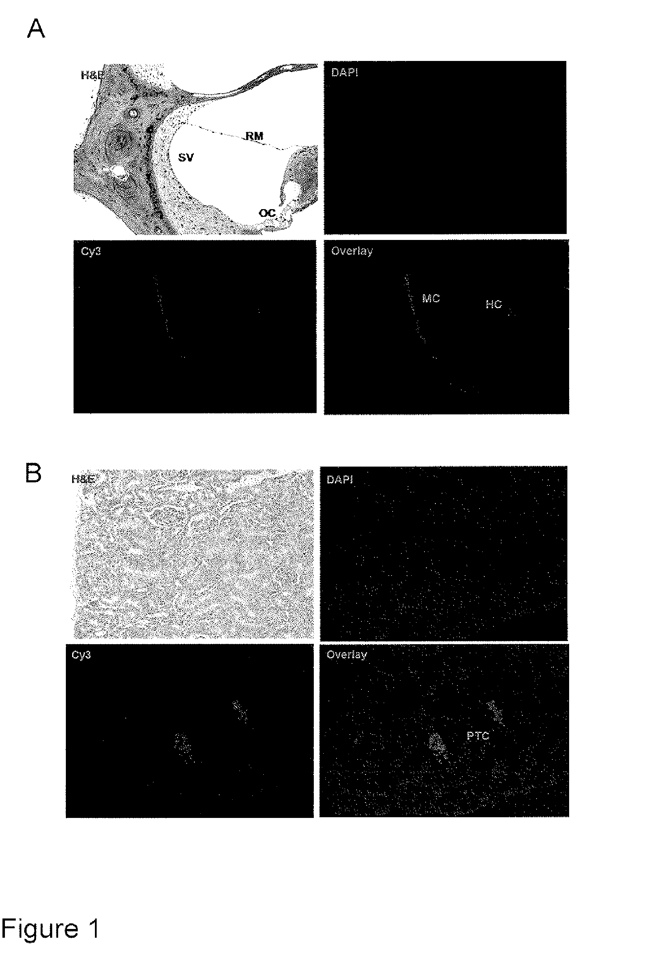

Figure 1 shows the high accumulation of platinum adducts in the DNA of particular cell types in the kidney and the cochlea of cis-platin treated mice. Platinum adducts forming interstrand crosslinks between adjacent guanines (Pt-GG) were visualized by immunostaining of frozen sections with the adduct specific antibody R-C18 (Cy3) and counterstaining of the nuclear DNA with 4',6-diamidino-2-phenylindole (DAPI). For morphological orientation serial sections of both organs were stained with hematoxyline and eosin (H&E; upper left micrographs). (A) Cochlea with high adduct levels in marginal cells (MC) in the stria vascularis (SV) and hair cells (HC) in the organ of Corti (OC). (B) Kidney with high adducts levels in proximal tubule cells (PTC). Magnification: 20x.

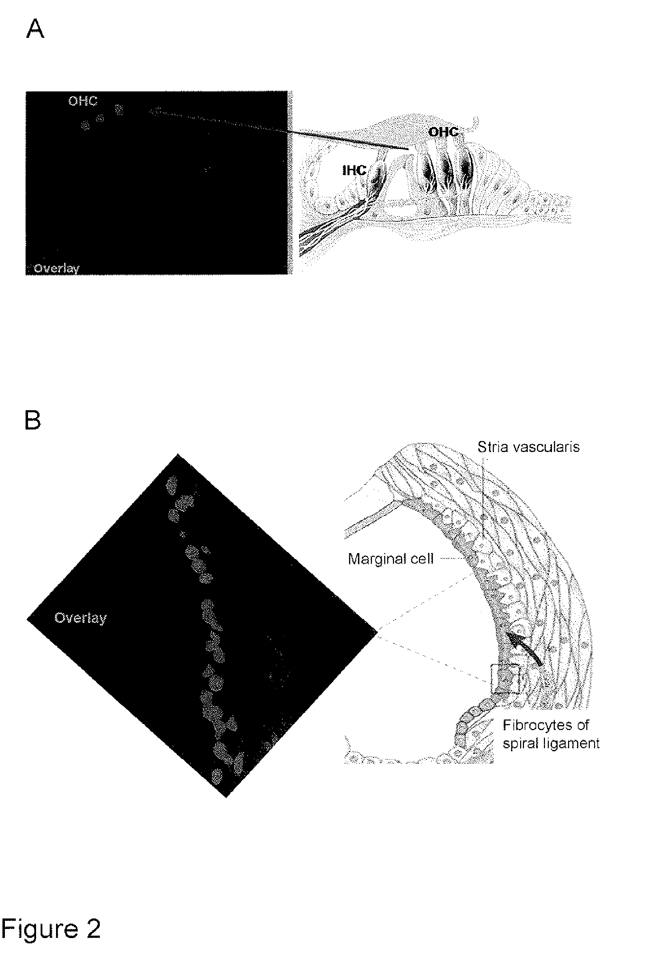

Figure 2 shows the localization of Pt-GG adducts in cells of the inner ear. Pt-GG intrastrand crosslinks in DNA were visualized by immunostaining of frozen sections with the adduct-specific antibody (Cy3) and counterstaining of the nuclear DNA with DAPI. (A) organ of Corti. Left: the nuclear DNA of the three outer hair cells is massively platinated (Magnification 60x). Right: schematic drawing of the organ of Corti with outer (OHC) and inner hair cells (IHC) (picture from Oxford II-lustrated Science Encyclopedia, modified). (B) Detailed view of the stria vascularis. Left: stria vascularis with highly platinated marginal cells (magnification: 60x). Right: schematic drawing of the stria vascularis with localization of marginal, intermediate and basal cells and fibrocytes of the spiral ligament (Jentsch 2000).

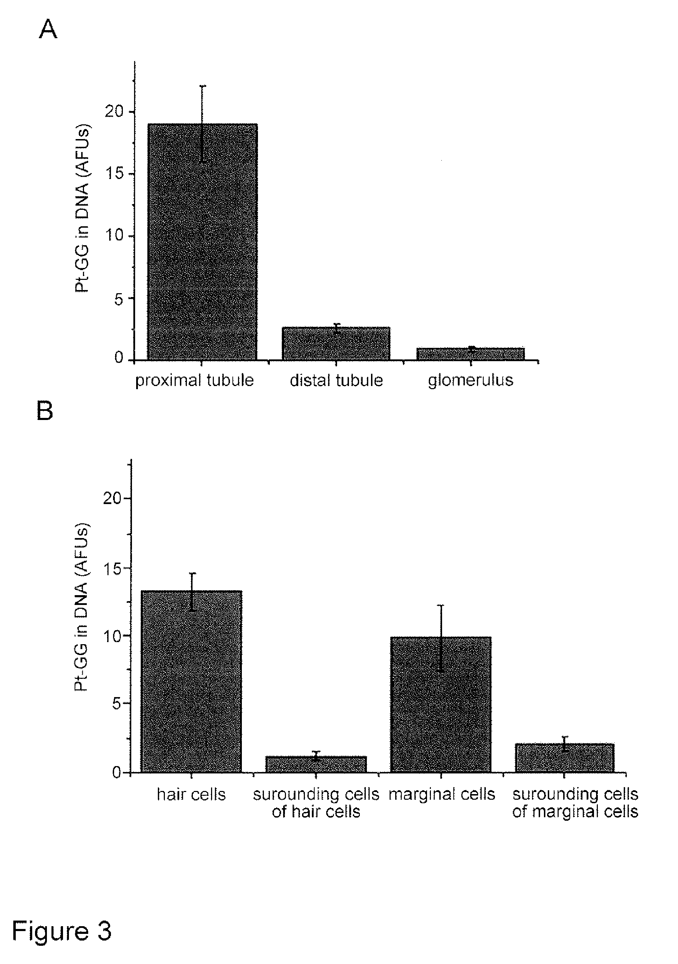

Figure 3 shows the accumulation of platinum-DNA adducts in different cell types of the cochlea and the kidney cortex 24 h after cis-platin treatment. (A) Distribution of Pt-GG adducts in the kidney cortex: Comparison between cells of the proximal or distal tubules and cells of the glomeruli. (B) Relative levels of Pt-GG adducts in outer hair cells (HC) and in marginal cells (MC) as compared to surrounding cells (SC). The columns represent mean values (+/- SD) from 100 measured cells (for HC from 50 cells). Levels of Pt-GG adducts were measured as Arbitrary fluorescence units (AFU).

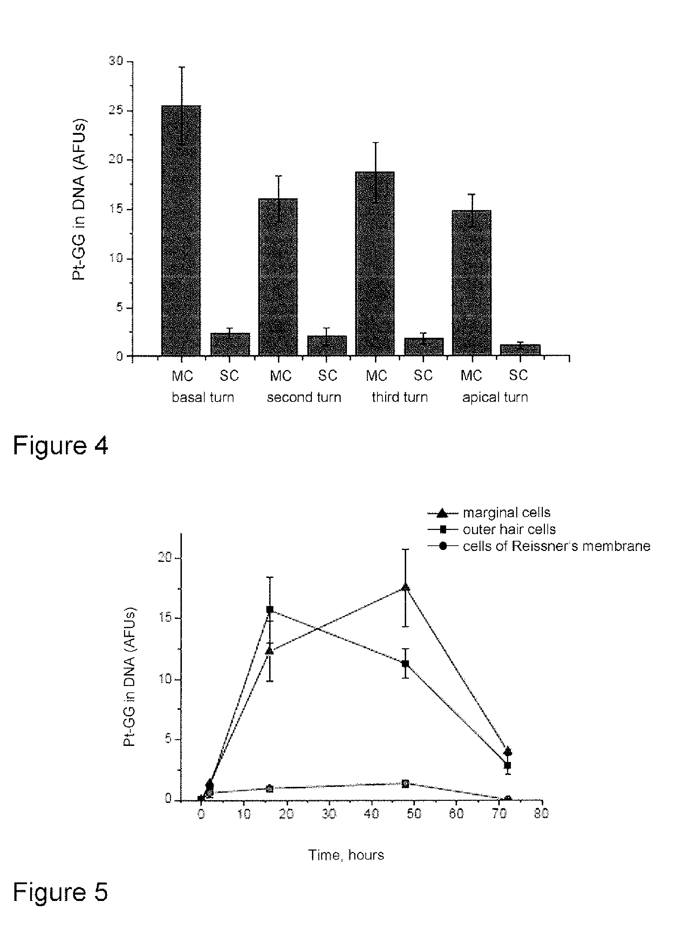

Figure 4 shows the levels of Pt-GG adducts in cells of the stria vascularis from different turns of the cochlea. Cochleae were dissected from mice 24 h after a single dose of cis-platin (10 mg/kg) and serial cryosections were measured for DNA adduct levels in different cell types by ICA analysis. Arbitrary fluorescence units (AFU) values were significantly higher in marginal cells (MC) of the basal turn as compared to the apical turn. A similar tendency was found for other cells of the stria vascularis (SC: intermediate and basal cells), however, on a 10-fold lower level of DNA platination.
Figure 5 shows the kinetics of Pt-GG adduct formation and repair in different cell types of the cochlea. Mice were treated with a single dose of cis-platin (12.5 mg/kg; i.p.) and were sacrificed at different time points. DNA adduct levels in outer hair cells, in marginal cells and in cells of Reissner's membrane were measured by immunocytological assay (ICA) analysis. The data represent mean values (+/-SD) of >50 individual nuclei per cell type and from 2 independent experiments.

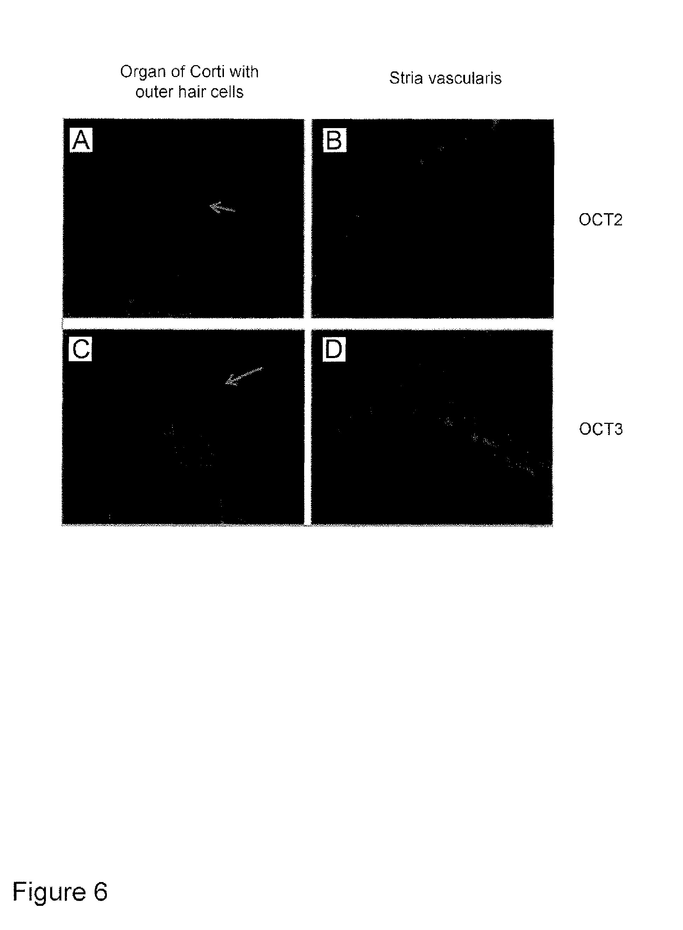

Figure 6 shows the immunohistochemical localization of organic cation transporters in the organ of Corti and the stria vascularis of the cochlea. Cryosections of formalin-fixed cochleae were immunostained (red) with antibodies for the membrane transporters organic cation transporter (OCT) 2 (A, B) and OCT 3 (C, D), and were counterstained for DNA with DAPI (blue). Arrows indicate the position of the outer hair cells in the organ of Corti. B and D: Cells of the stria vascularis with OCT 2 and OCT 3 located in the area of the intermediate cells. (Magnification: A: 60x; B, C, D: 40x).

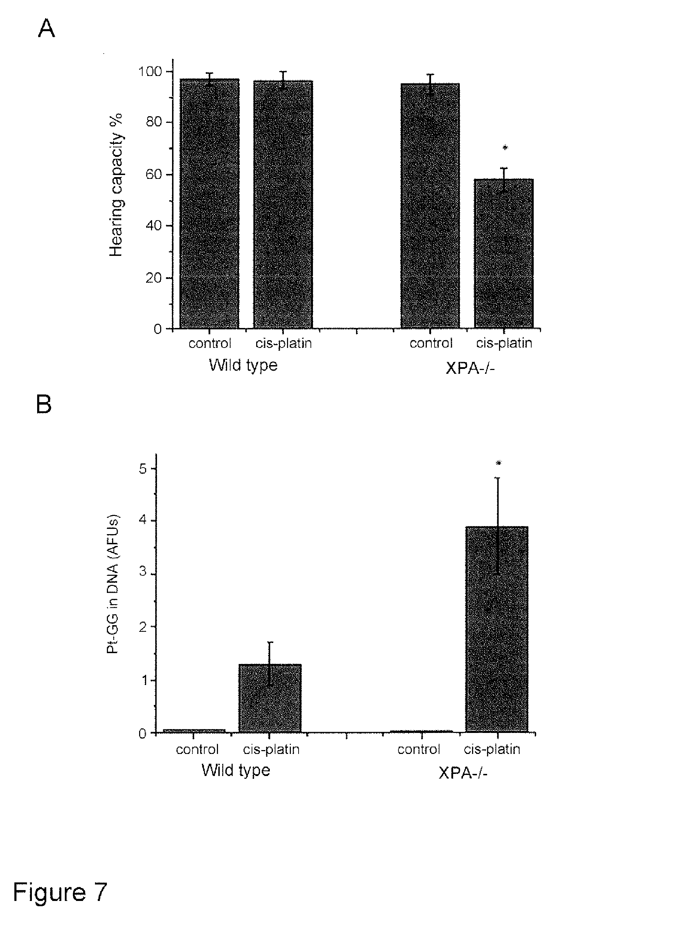

Figure 7 shows that mice with reduced DNA repair capacity are more prone to cis-platin induced ototoxicity and accumulate higher levels of Pt-GG adducts in the DNA of cochlear target cells. C57BL/6 (wild type) mice and syngenic DNA repair-deficient (XPA-/-) mice were repetitively treated with low doses of cis-platin (cumulated dose: 3.5 mg/kg) and their hearing capability was measured 24 h after the last dose by the BAER test in comparison to untreated controls. (A) The columns represent means (+/- SD) of three consecutive measurements per animal and of 5 mice per treatment group. The hearing capability was significantly reduced by 45 % in the group of cis-platin-treated XPA-/- mice whereas no effects were observed in untreated XPA-/- mice or in wild type mice after cis-platin exposure. (B) Levels of accumulated DNA adducts in the inner ear. All mice in the experiment were sacrificed immediately after the BAER test and cryosections of cochlea were analyzed for Pt-GG adduct levels by ICA analysis. After equal doses of cis-platin, inner ear cis-platin target cells of XPA -/- mice depicted more than 3 times higher adduct levels than those of the wild type littermates. * indicates *P* ≤ 0.001 vs. wildtype.

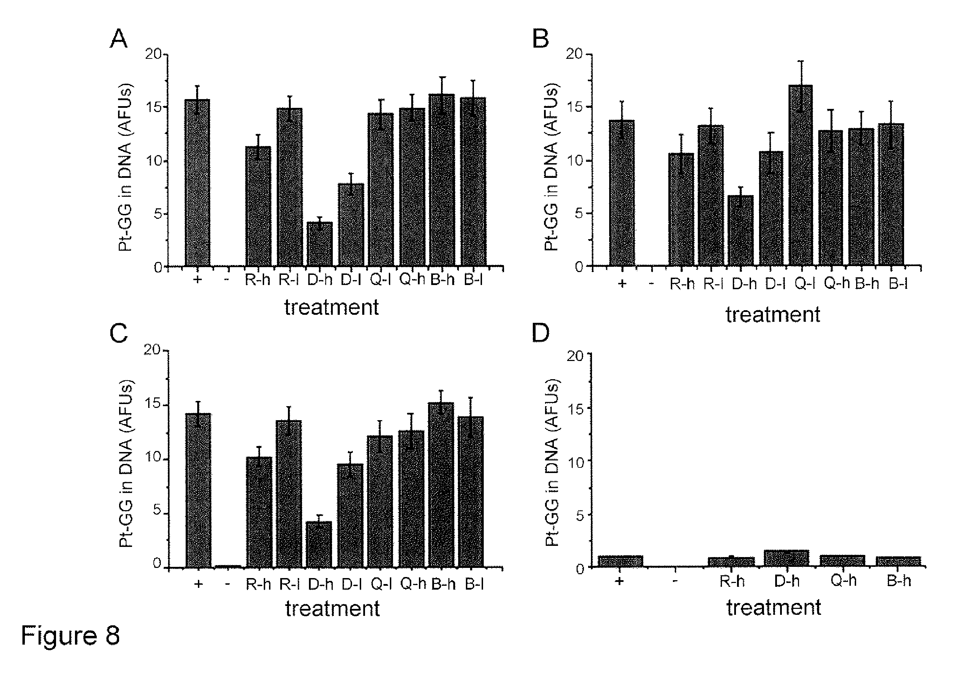

Figure 8 shows the accumulation of the Pt-GG adducts in cells of the kidney and the inner ear of cis-platin treated mice with co-administration of transport inhibitors. Potential inhibitors were injected i.p. at two different doses 1 h before cisplatin treatment (10 mg/kg). Mice were sacrificed 24 h after cis-platin treatment and Pt-GG adducts were measured in the DNA of target and non-target cells by ICA analysis. (A) Proximal tubule cells of the kidney. (B) Outer hair cells of the cochlea. (C) Stria vascularis marginal cells of the cochlea. (D) Cells of Reissner's membrane. (+): cis-platin; ( - ): PBS treated control; R: Ranitidine, D: Diphenhydramine; Q: Quinine; B: Butylscopolamine; h: high dose; I: low dose.

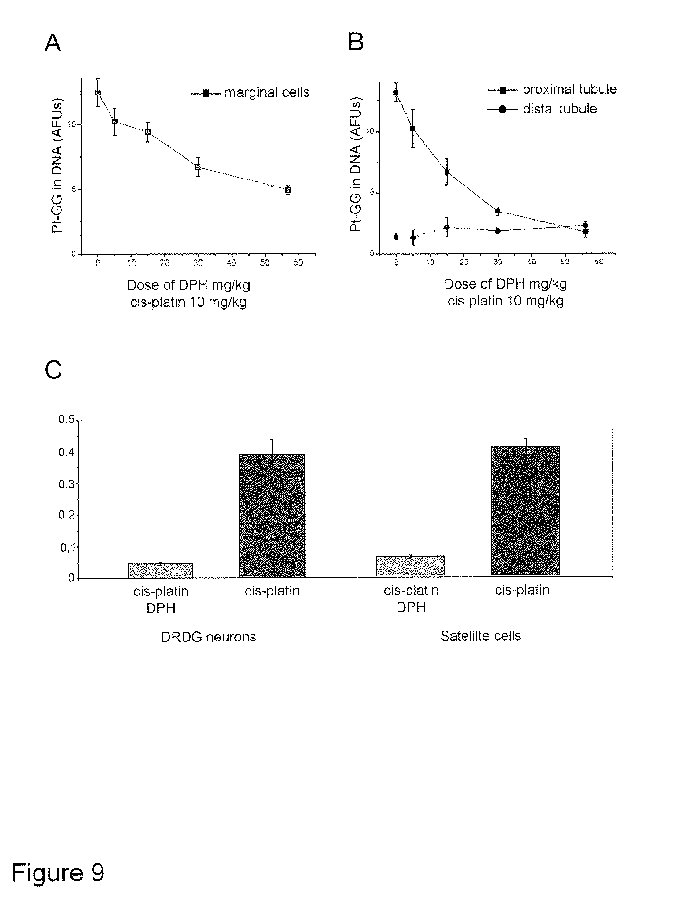

Figure 9 shows the dose-dependent effect of DPH on Pt-adduct accumulation in different cell types. Mice were treated with cis-platin alone (0) or in combination with different doses of DPH (5, 15, 30, 57 mg/kg), sacrificed 24 h later and the DNA Pt-adduct levels were measured in kidney and cochlear cells by ICA analysis. Figure 9C shows the accumulation of platinum-DNA adducts in dorsal root ganglion (DRG) neurons and their satellite cells. Levels of Pt-adducts were analyzed 24 h after administration of cis-platin (15 mg/kg) alone or in combination with DPH (50 mg/kg).

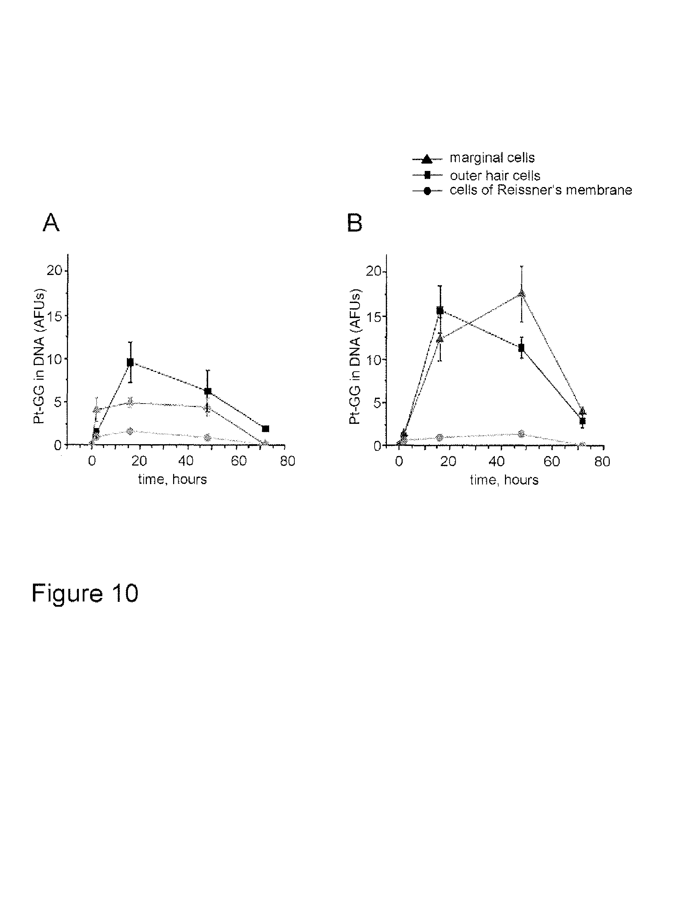

Figure 10 shows the influence of DPH co-application on the formation and repair kinetics of Pt-GG adducts in the DNA of different cochlear cell types of cis-platin treated mice. Animals were pre-treated or not with DPH (57 mg/kg) and were i.p. injected 30 minutes later with cis-platin (12.5 mg/kg). Each two mice were sacrificed at 2, 16, 48, or 72 hours after cis-platin and Pt-GG adduct levels in the DNA of inner ear cells were determined by ICA analysis for outer hair cells, marginal cells and of Reissner's membrane. (A) With DPH. (B) Without DPH.

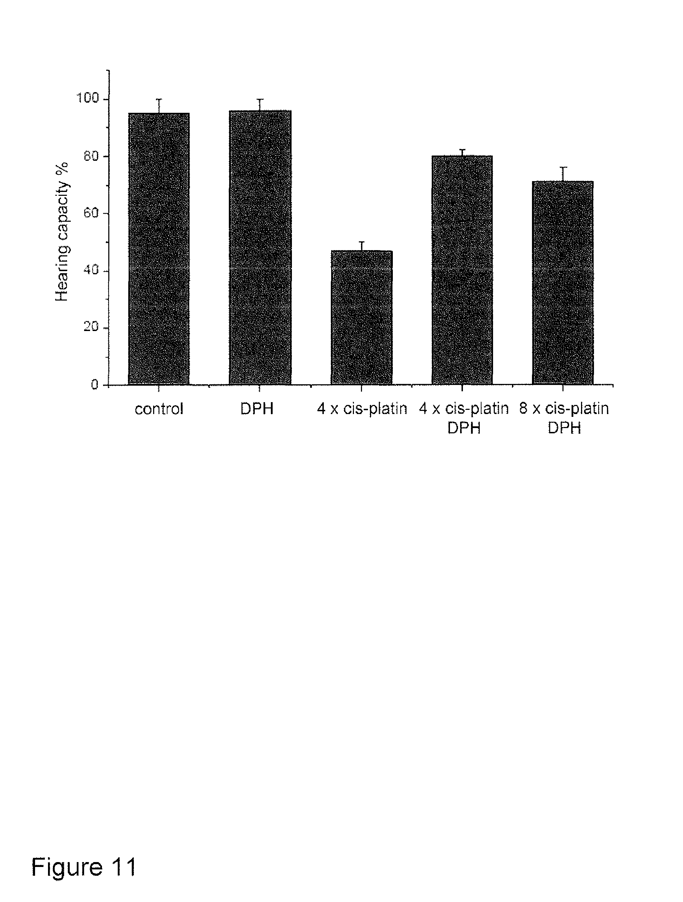

Figure 11 shows the protective effect of DPH on the hearing capability of cis-platin treated mice. To induce functional loss in the hearing system mice were treated repetitively 4 times with cis-platin alone (5 mg/kg every second day, cumulated dose: 20 mg/kg) or with concomitant injections of DPH (57 mg/kg) 30 minutes before each dose of cis-platin. Additional groups were injected accordingly only with PBS or only with DPH or were treated with cis-platin + DPH eight times (cumulated dose of cis-platin: 40 mg/kg). All animals were measured for their hearing capability with the BAER test 24 h after the last dose of cis-platin was applied. The columns represent mean values (+/- SD) from five animals per treatment group and were calculated as percentage of the control group.

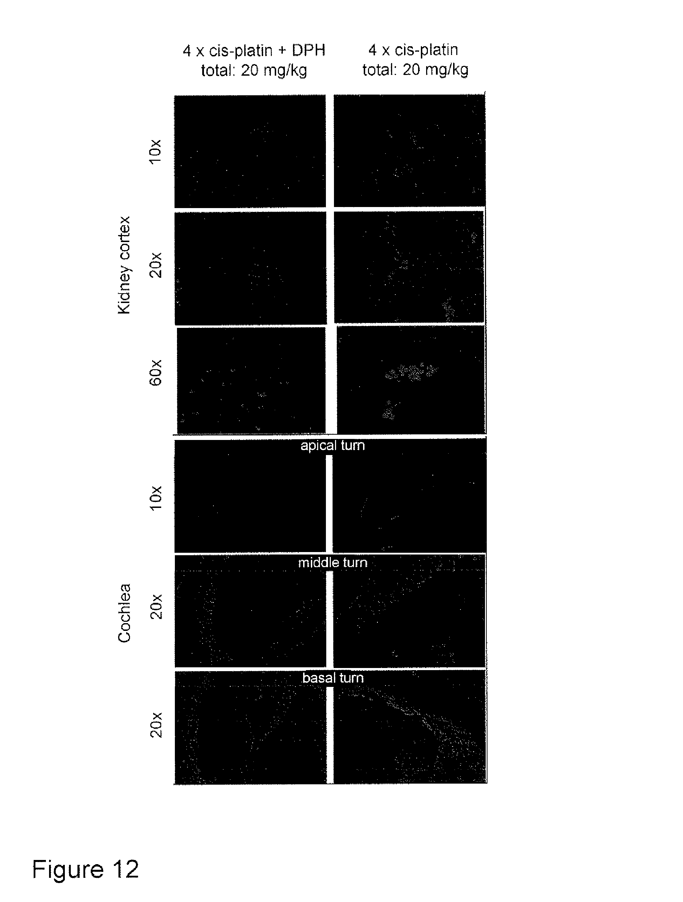

Figure 12 shows the distribution of platinum adducts between different cells of the kidney cortex and the chochlea after repetitive cis-platin and cis-platin plus DPH treatment, respectively. Mice were treated repetitively 4 times with cis-platin alone (5 mg/kg every second day, cumulated dose: 20 mg/kg) or with concomitant injections of DPH (57 mg/kg) 30 minutes before each dose of cis-platin. Animals were sacrificed after the last BEAR test and Pt-GG levels in the DNA of kidney cells and chochlea cells were visualized by immunostaining; DAPI - DNA.

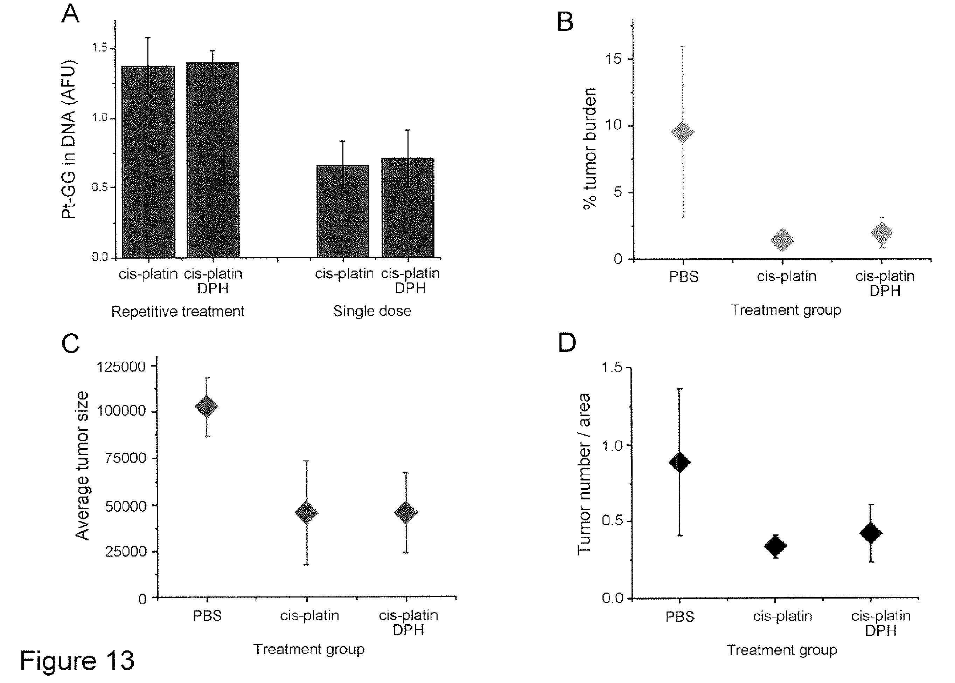

Figure 13A shows the accumulation of platinum-DNA adducts in primary mouse lung tumors during acute and chronic treatment by cis-platin or cis-platin plus DPH. Animals were sacrificed 24 h after the last drug injection and lung cryosections were measured by ICA analysis. The two left columns represent mean adduct levels (+/- SD) in lung tumors cells of mice treated with cis-platin four times (5 mg/kg, every second day) with (cis+DPH) or without (cis) co-application of DPH (57 mg/kg). The two right columns refer to the respective adduct levels after a single high dose of cis-platin (10 mg/kg).
Figure 13 B - D show that the co-application of DPH had no inhibitory effect on the therapeutic efficacy of cis-platin on primary lung tumors in mice. Four tumor-bearing animals were treated with cis-platin alone or in combination with DPH or were mock-treated with PBS. H&E-stained lung sections were analyzed quantitatively for the percentage of tumor burden (B), the average tumor size (C), and the number of tumors per section (D) by using Bioquant image analysis software. For none of these parameters a significant alteration was observed when comparing cis-platin and cis-platin plus DPH treatment schedules.

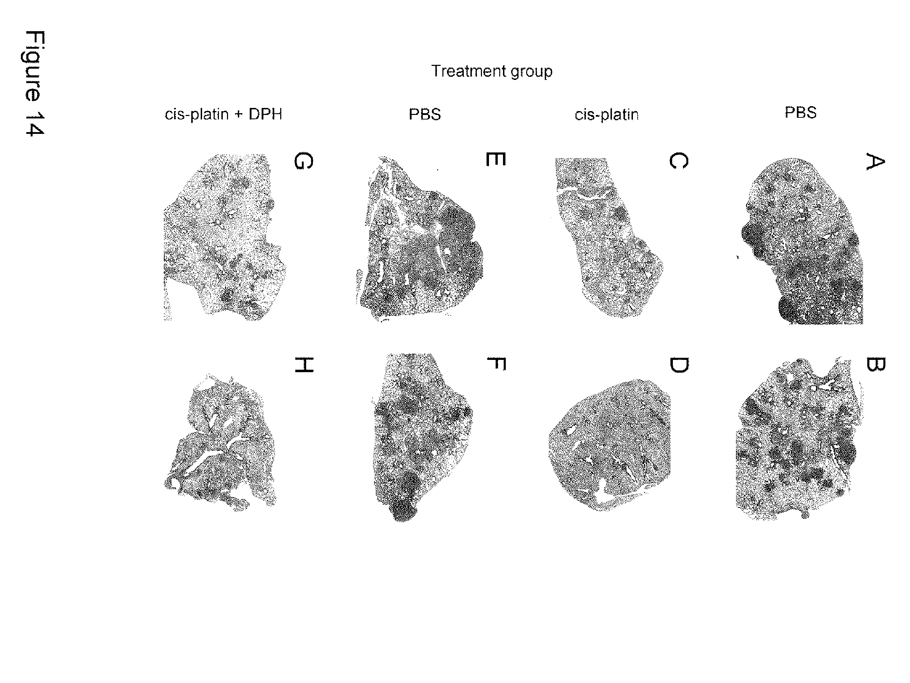

Figure 14 shows representative photographs of the H&E stained lung sections from tumor-bearing mice from three treatment groups. Twelve weeks after tumor initiation mice were treated with cis-platin alone (7 mg/kg once a week for 4 weeks; C, D) or in combination with DPH (57 mg/kg; G, H)) or were mock-treated with PBS (A, B, E, F). Five days after the last treatment lungs were resected, fixed in formalin and embedded in paraffin. Morphological structures were visualized in tissue sections (6 µm) by H&E staining (magnification: 2x). Tumor areas are stained dark purple.

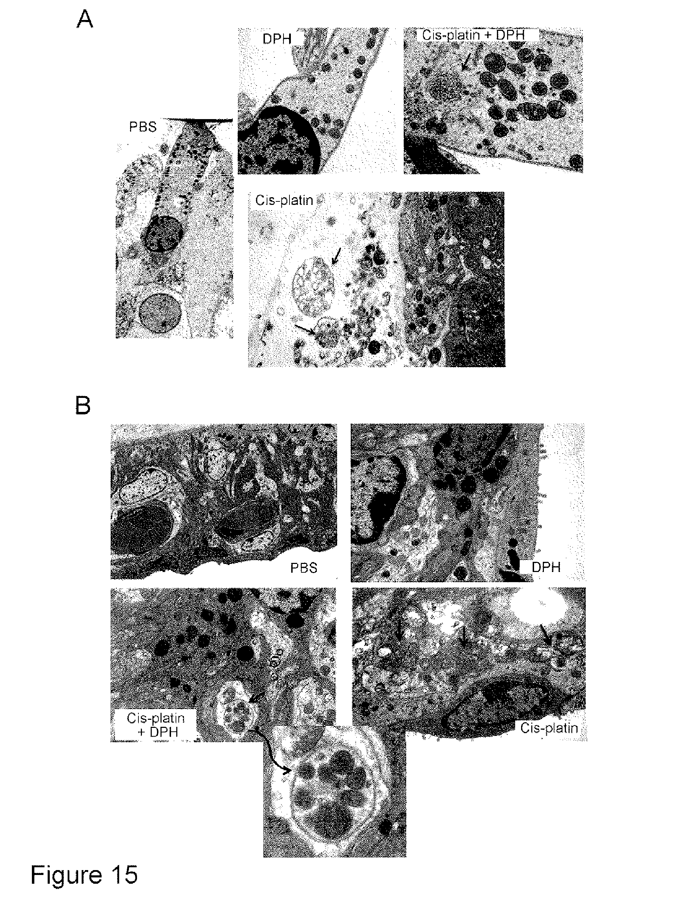

Figure 15 shows micromorphology of outer hair cells (A) and stria vascularis marginal cells (B) in the cochlea of mice repetitively treated every second day for four times with PBS, or with DPH (57 mg/kg), or with cis-platin (5 mg/kg) or with both drugs. Cells were visualized in ultrathin sections by electron microscopy. The morphology of the cytoplasm was normal without any signs of autophagy in PBS-as well as in only DPH-treated mice. Cochleae from mice treated with cis-platin plus DPH depicted rare autophagosome-like structures (arrow) in the cytoplasm. Treatment with cis-platin alone caused necrotic cell death as indicated by incomplete and lumpy autophagosomes (arrow) as well as cellular swelling, and scattered chromatin condensation.

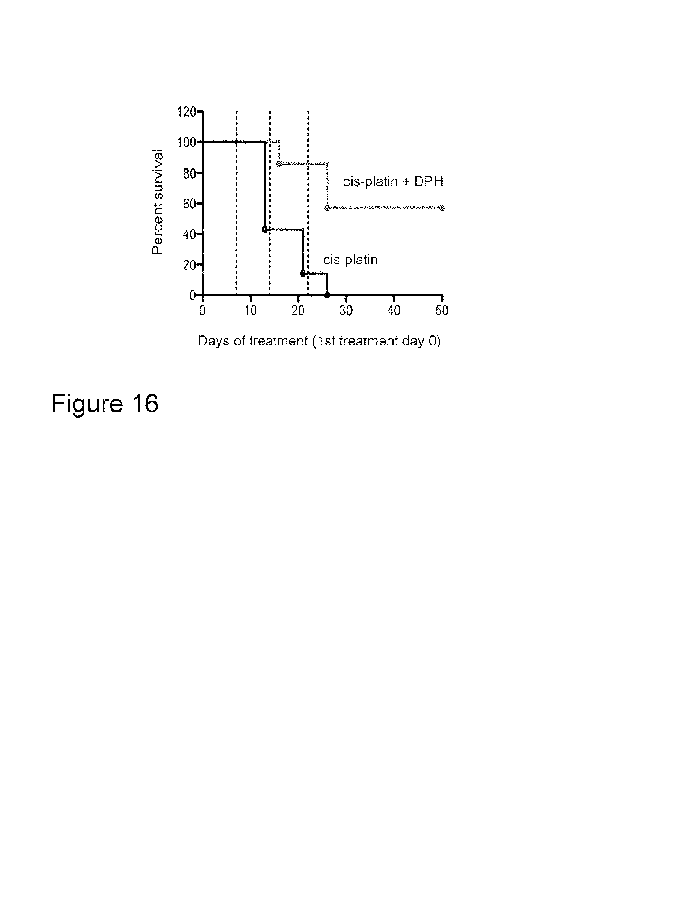

Figure 16 shows survival rates of Kras/p53 deficient mice upon repetitive treatment with cis-platin (9 µg/g = 9 mg/kg) alone or in combination with DPH (50 µg/g = 50 mg/kg).

### Detailed description of the invention

In a first aspect, the invention is directed to diphenylhydramine (DPH) for treating ototoxicity in a subject, wherein the ototoxicity is induced by a medication.

The term "diphenylhydramine" comprises compounds of the formula I 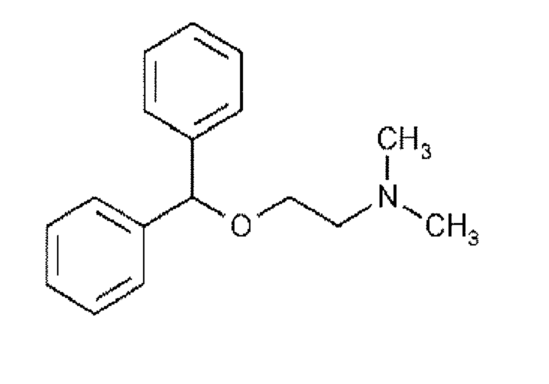 and related compounds as well as derivatives and pharmaceutically acceptable salts thereof. Thus, the term also includes compounds which have chemical moieties that deviate from formula I, but the compound shows similar or identical chemical/or pharmaceutical properties as the compound of formula I. DPH was originally found to have antihistaminic properties but is nowadays used to treat a variety of conditions including allergic symptoms, motion thickness, insomnia and extrapyramidal symptoms. Although it has been found that DPH executes its antihistaminic function through inhibiting the histamine H₁ receptor, relatively little is known about the mechanism of the other diverse effects of DPH.

The term "treating" or "treatment" as used herein includes preventing a disease, *i.e.*, causing the clinical symptoms of the disease not to develop in a subject, inhibiting the disease, *i.e.*, arresting or reducing the development of the disease or any of its clinical symptoms, and relieving the disease, *i.e.*, causing regression of the disease or any of its clinical symptoms.

The term "medication" as used herein refers to any substance used during medical treatment, therapy, prevention and/or diagnosis and means the same as pharmaceutical drug, medicine or medicament.

The term "ototoxicity" as used herein refers to the functional impairment and/or cellular degradation of the tissue of the inner ear manifesting as sensorineural mono- or bilateral hearing loss, and/or transient or permanent tinnitus. Many medications, including antibiotics, chemotherapeutic agents, diuretics and analgesics induce ototoxic side effects. Although ototoxicity only occurs in a low frequency for most medications, it is considered a serious side effect, since it can lead to irreversible damages or even total deafness. In addition, some medications, in particular cancer medications, show ototoxic side effects in most patients. Using cis-platin as a model for a medication inducing ototoxicity, the inventor found that the application of DPH counteracts the ototoxic side effect. Applying cis-platin to mice severely impaired their hearing capacity as determined using brain stem auditory evoked response (BAER), a clinical screening test to monitor for hearing loss and deafness assessing the functions of the ears, cranial nerves and various brain functions of the lower part of the auditory system. The examination revealed that the cis-platin treatment reduced hearing capacity by about 45 %. Interestingly, the inventor found that this effect could be prevented by administrating DPH together with cis-platin, maintaining the hearing capacity at more than 80 %. Thus, besides its other functions DPH is suitable to inhibit the occurrence of hearing loss induced by ototoxic medication. Accordingly, a method for treating ototoxicity in a subject is disclosed, comprising administering DPH, wherein the ototoxicity is induced by a medication.

In a preferred embodiment, the medication comprises an anti-cancer agent, preferably a platinum complex, a nitrogen mustard derivative or vincristine; an antibiotic, an ototoxic diuretic and/or a quinine derivative. Several anti-cancer drugs, as e.g. cis-platin, nitrogen mustard, and vincristine, as well as antibiotics, e.g. capreomycin, gentamicin, dihydrostreptomycin and neomycin, and diuretics as e.g. furosemide, bumetanide and ethacrynicacid, have been shown to induce ototoxicity. Likewise, quinine derivatives as e.g. quinidine, quinacrine and mefloquinine have been reported to cause tinnitus and hearing loss.

Of the cancer drugs known to cause ototoxic side effects, cis-platin is most commonly used. It is usually applied for treating solid tumors of epithelial origin, as e.g testicular cancer, ovarian, cervical, bladder and non small cell lung carcinoma as well as head and neck cancer and medulloblastoma and osteogenic sarcoma. Cisplatin is a small uncharged platinum complex having a molecular weight of about 300 g/mol with two chloride ligands. Although the compound is rather stable as long as it remains in a high chloride environment, such as the blood stream, its ligands become sequentially exchanged by water molecules once cis-platin enters the cellular plasma having chloride concentrations as low as about 5 mM. By exchanging the chloride ligands by water molecules highly reactive positively charged aqua complexes are formed which are either monohydrated [Pt(NH)Cl(OH)] or dehydrated [Pt(NH)OH)]. The positively charged hydrated forms of cis-platin are electrostatically attracted by negatively charge macromolecules in the cell such as DNA, RNA or proteins. When binding to DNA, cis-platin facilitates reactions with nucleophilic centers like the N7 atom of guanine. Due to the presence of two potentially reactive sides, cis-platin forms crosslinks within DNA strands, thereby intercalating into the DNA molecule. These intercalations are termed "platinum adducts" (Pt-adducts) and cause chemical modifications altering the structural integrity of the DNA molecule. Among Pt-adducts, the most frequent structures are interstrand crosslinks between adjacent guanines (Pt-GG) and crosslinks between neighboring adenine and guanine bases (Pt-AG). These Pt-adducts, together with interactions of cis-platin with RNA, and proteins involved in cell signaling and cell metabolism, are believed to cause the induction of apoptosis in tumor cells and thus to be responsible for the anti-neoplastic effect of cisplatin in cancer therapy.

The Pt-adducts formed by cis-platin and two adjacent guanines within the DNA lead to a significant bending towards the major groove of the helix and in turn expose the minor groove of the helix. This can be detected using a Pt-GG specific monoclonal antibody (R-C18, Liedert *et al.*, 2006). Using this antibody, the inventor found specific accumulations of Pt-adducts in cells of the inner ear. This suggests that the formation of Pt-adducts causes the structural damages observed in the inner ear upon administration of cis-platin, with loss of cochlear hair cells beginning from the basal turn and degeneration of the stria vascularis and the spinal ganglion neurons. Consistently with the observed reduced hearing damage upon the application of DPH, the inventor also found that DPH decreased the cis-platin induced formation of Pt-adducts in the cells of the inner ear.

In a preferred embodiment, the platinum complex is selected from the group consisting of cis-diaminedichloro-platinum(11) (cis-platin), cis-platin analogs, amine platinum complexes, diamine platinum complexes, trans-platinum complexes multinuclear platinum complexes and Platinum(IV) coordination complexes. Besides cis-platin, also novel derivatives having similar but more efficient anti-neoplastic functions are expected to cause ototoxic side effects as cis-platin.

In a preferred embodiment, the medication is administered during a cancer treatment. Ototoxicity is often observed as a side effect upon administration of a medication in high doses and for a long period. Such therapeutic regimes are usually necessary for cancer treatment to possibly remove the cancer cells entirely. In cancer cells, the natural cellular mechanisms leading to apoptosis upon severe DNA damages are disturbed, making the cells difficult to eliminate. Moreover, due to accelerated and uncontrolled proliferation few surviving cancer cells can sufficiently repopulate, such that the cancer reoccurs. Therefore, cancer treatments are particularly aggressive to possibly remove all cancer cells from the patient's body. Since DPH counteracts the drugs ototoxic side effects, higher and therefore more efficient dosages of anti-cancer drugs may be administered in its presence. Accordingly, a method for treating ototoxicity in a subject is disclosed, comprising administering DPH to the subject, wherein the subject receives a cancer medication.

In a further preferred embodiment, the cancer is a cancer hypersensitive to a platinum complex. Although DPH inhibits the formation of Pt-adducts in cells of the inner ear, it does not interfere with the anti-neoplastic effects of a platinum-based anti-cancer drug as cis-platin. Administering cis-platin along with DPH to mice bearing K-ras^{S12D} induced tumors resulted in the inhibition of Pt-adduct formation in cells of the inner ear but not in tumor cells. In fact, the application of DPH did not attenuate the reduction in tumor size and number due to cis-platin. The administration of cis-platin alone as well as the application of cis-platin in combination with DPH reduced the tumor burden by about 80 % and the average tumor size by about 50 %. Thus, the application of DPH is particularly advantageous in combination with a medication directed to a platinum sensitive cancer. The sensitivity of the cancer to a platinum complex may be natural or induced by a drug and/or a chemotherapeutic regime.

In a preferred embodiment, the sensitivity is induced by a drug and/or a chemotherapeutic regime. Medication induced ototoxicity often prevents the use of platinum-based anti-cancer drugs. Thus, by counteracting this side effect through administering DPH, the scope of platinum-based medications, in particular platinum-based anti-cancer drugs can be significantly broadened. Besides higher and more concentrated dosage regimes the application of platinum-based cancer medications may be expanded by inducing a platinum sensitivity in cancer cells that are not naturally sensitive to platinum. The sensitivity may be induced by drugs and/or chemotherapy regimes.

In a preferred embodiment, the cancer is selected from the group consisting of testicular cancer, ovarian cancer, cervical cancer, bladder cancer, small and non small cell lung cancer, mammary cancer, prostate cancer, pancreatic cancer, head and neck cancer, melanoma, neuroblastoma, soft tissue cancer, medulloblastoma and osteogenic sarcoma. These types of cancers have been efficiently treated using cis-platin. However, the use of this promising medication is often limited by the occurrence of hearing damages due to the ototoxic properties of cis-platin. Efficiently counteracting these side effects with DPH, in particular in combination with increasing the platinum sensitivity of cancer cells, makes platinum-based cancer treatments available for an increased number of patients.

In a preferred embodiment, DPH is administered to the subject before or concurrently with the medication. For preventing ototoxicity, it is preferred to administer DPH 0 to 45 minutes, preferably 0 to 30 minutes, more preferred 0 to 15 minutes before the ototoxic medication such that DPH is already distributed throughout the patient's body including the cells of the inner ear, when the medication is administered. Alternatively, DPH may be applied together with the medication such that both compounds distribute simultaneously. Thus, the application of DPH may easily be integrated into cancer treatments which usually comprise carefully planned therapeutic regimes. For counteracting ototoxicity of a medication, DPH may also be applied after the medication has already been administered. This is particularly advantageous for medications which only occasionally show ototoxic side effects. In this case DPH can be applied as soon as the first symptoms of hearing damage arise. By this the damages may be remedied and further health deterioration prevented without exposing the patient to further chemicals *a priori*.

In a preferred embodiment, DPH is administered in a dosage, which correlates with the dosage of the medication. In general, efforts are made to increase dosages of medication as much as possible to achieve most efficient treatments. This is often prevented by side effects which usually intensify with increasing dosages of medications. The inventor found that DPH can repress ototoxic side effects even at high doses of cis-platin by increasing the dosage of DPH. Likewise, he found that reduced dosages of DPH are sufficient to inhibit ototoxicity induced by low dosis of cis-platin. Thus, the dosage of DPH may be adapted to the dosage of the ototoxicity inducing medication without loosing efficiency. This means that the dosage of DPH can be minimized according to the dosage of the ototoxic medication, thereby reducing the physical strain for the patient as much as possible. On the other hand, ototoxicity resulting from high doses of medications can be prevented by using correlating doses of DPH.

In a preferred embodiment, DPH is used for additionally treating nephrotoxicity. Besides ototoxicity, nephrotoxicity is a commonly observed side effect of many medications. This is because urinary excretion is the main route for removing drugs from the organism. Many drugs are freely filtered at the glomeruli and taken up into the kidney cells which reabsorb filtered physiological ions by active transport. This leads to an undesired accumulation of pharmaceutical compounds in the kidney cells impairing their health, sometimes even causing extensive cell death which may lead leads to renal failure. Surprisingly, the inventor found that DPH does not only counteract medication induced ototoxicity but also nephrotoxicity. Since cis-platin treatment causes structural damages of kidney cells, in particular of proximal tubular cells, and increases urinary creatinine values together with a failure of magnesium, sodium and potassium reabsorption, the inventor used cis-platin treated mice as a model system for medication induced nephrotoxicity. Administering DPH before cis-platin revealed reduced damages of proximal tubular cells in comparison to administering cis-platin alone. Thus, DPH is well suited for concurrently treating ototoxicity and nephrotoxicity, which often occur together as medication induced side effects. This reduces the number of drugs to be administered to a patient for counteracting undesired side effects which particularly often and severely occur for cancer treatments.

In a further aspect, the invention is directed to DPH for inhibiting the formation of platinum adducts in a cell of the inner ear. The inventor found that the administration of cis-platin leads to an accumulation of Pt-adducts in cells of the inner ear. These caused cellular damages leading to the degradation of hair cells and marginal cells, both participating in the highly sophisticated system of different cellular and endolymph ion concentrations in the inner ear. This system of ion concentrations is essential for the translation of sound into neuronal signals, such that damages to the system manifest in impaired hearing or even deafness. Besides a loss of hair and marginal cells, the accumulation of Pt-adducts also causes a reduction of the membrane potential of outer hair cells resulting in a weakened respond to sound pressure. The inventor further found that these structural and functional damages of cells of the inner ear are prevented by the administration of DPH. Accordingly, in a further aspect, the invention is directed to DPH for preventing and/or inhibiting structural and functional damage in the inner ear during a platinum-based treatment.

In a preferred embodiment, the cell of the inner ear is a cell of the organ of Corti, preferably an outer hair cell or a cell of the stria vascularis, preferably a marginal cell. Using an antibody specifically directed against Pt-GG adducts, the inventor revealed that unexpectedly the Pt-adducts were not widely and evenly distributed throughout the cells of the inner ear, but accumulated in specific cell types of two particular areas, namely the organ of Corti and the stria vascularis. High levels of Pt-adducts were exclusively found in outer hair cells but not in inner hair cells of the organ of Corti and in the outer cell layer, i.e. in the marginal cells of the stria vascularis but not in neighboring intermediate or basal cells. The administration of DPH along with cis-platin inhibited the formation of Pt-adducts in these cells by about 70 %.

In a further aspect, the invention is directed to an agent reducing the import of a platinum complex into a cell and/or increasing the export of a platinum complex from a cell, preferably a post-mitotic cell, for treating ototoxicity, wherein the ototoxicity is induced by a medication comprising a platinum complex. The specific accumulation of Pt-adducts in marginal cells and outer hair cells is further remarkable, because these cells are post-mitotic, in contrast to cancer cells which are believed to accumulate cis-platin due to their excessive proliferation. This leads to the notion that the particular high levels of Pt-adducts in marginal cells and outer hair cells are based on a molecular mechanism, which is specific for these types of cells. The inventor could successfully show that marginal cells and outer hair cells display similar kinetics of Pt-adduct formation and repair compared to other cells of the inner ear, suggesting that the accumulation of Pt-adducts is due to an increased uptake or reduced export of platinum complexes into the cells. Accordingly, a method is disclosed for treating ototoxicity induced by a medication comprising a platinum complex in a subject, comprising administering to the subject an agent reducing the import of the platinum complex into a cell and/or increasing the export of a platinum complex from a cell, preferably a post-mitotic cell.

In a further aspect, the invention is directed to an agent for treating ototoxicity induced by a platinum complex, wherein the agent interferes with a modulator of the import and/or the export of the platinum complex into/from a cell. By changing the expression or the activity of a molecule which regulates the import of platinum complexes into a cell, the accumulation of Pt-adducts in the cell can be prevented. Moreover, by increasing the expression or the activity of a molecule regulating the export of platinum complexes from a cell the removal of platinum complexes from the cell can be improved thereby reducing the intracellular platinum concentration and preventing the accumulation of Pt-adducts. Accordingly, a method for treating ototoxicity in a subject induced by a medication comprising a platinum complex is disclosed, comprising administering to the subject an agent, which interferes with a modulator of the import and/or export of the platinum complex into/from a cell. An agent specifically interfering with such a transporter molecule exerts little effect on the surrounding cells as the transporter is specific for marginal cells and outer hair cells. In addition, the uptake of platinum complexes into cancer cells does not depend on a specific transport mechanism, and cancer cells were not found to actively export platinum complexes, such that the agent does not interfere with the anti-neoplastic effects of the platinum complex.

In a preferred embodiment, the agent inhibits an endogenous transporter of the platinum complex, wherein the transporter imports the platinum complex into the cell. For maintaining the sophisticated homeostasis of ion concentrations in the inner ear, marginal cells and outer hair cells express particularly many and diverse transporter molecules. One or more of these may actively transport platinum complexes into the cells cytoplasm. Such transporters may be transmembrane molecules which are expressed in high amounts in marginal cells and outer hair cells.

In a preferred embodiment, the agent inhibits the expression or activity of the transporter. This prevents or interrupts the import of platinum complexes into the cells. In addition, biological systems that depend on narrowly balanced concentrations of compounds such as hormones, proteins or ions, as for example the homeostasis of the inner ear, are often maintained by negative and/or positive feedback mechanisms. Thus, the expression levels as well as the activity of transporter molecules are believed to be regulated by the presence and/or concentration of endogenous compounds. Such compounds as well as their derivatives can provide effective agents for decreasing the expression and/or activity of transporter molecules responsible for the import of platinum adducts into a cell.

In an alternative embodiment, the agent promotes an endogenous transporter of the platinum complex, wherein the transporter exports the platinum complex from the cell. One or more of the transporter molecules expressed by marginal cells and outer hair cells may actively export platinum complexes from the cell's cytoplasm. Their specific stimulation by an agent can be used to actively remove platinum complexes from the cells of the inner ear.

In a preferred embodiment, the agent promotes the expression or activity of the transporter. This increases the export of platinum complexes from the cells, thereby preventing their accumulation within the cell's cytoplasm. In addition endogenous compounds can provide efficient agents for increasing the expression and/or activity of transporter molecules responsible for the export of platinum adducts from a cell.

In a particularly preferred embodiment the cell is a cell of the inner ear, preferably of the organ of Corti or the stria vascularis. By providing an agent that interferes with a transporter molecule specific for cells of the inner ear, preferably marginal cells and/or outer hair cells, the accumulation of Pt-adducts is specifically inhibited in these cells. Thus, ototoxicity is prevented, without affecting other cells of the patient's body that do not express the respective transporter molecule.

In a further aspect, the invention is directed to DPH for treating nephrotoxicity in a subject induced by a medication comprising a platinum complex, wherein DPH inhibits the formation of platinum adducts within a cell of the kidney, preferably of the kidney cortex, more preferred a proximal tubular cell. Platinum comprising drugs, such as cis-platin, show nephrotoxic side effects that lead to irregularities in nutrient and ion reabsorption and, in severe cases, to renal insufficiency. The inventor found that these effects of platinum-based medications are due to accumulation of Pt-adducts in specific cells of the kidney, namely in the proximal tubular cells. In contrast, other cell types of the kidney even in immediate proximity of the proximal tubules do not show elevated Pt-adduct levels. The accumulation of the Pt-adducts leads to structural damages and loss of proximal tubular cells and causes the renal dysfunction regularly observed upon cis-platin treatment. Importantly, the inventor could show that this accumulation of Pt-adducts and the resulting cell death is prevented by the application of DPH. DPH counteracts the accumulation of Pt-adducts and thereby protects the proximal tubules from damages and cell death. Accordingly, a method for treating nephrotoxicity in a subject induced by a medication comprising a platinum complex is disclosed, comprising administering DPH to the subject, wherein DPH inhibits the formation of platinum adducts within kidney cells.

In a preferred embodiment, the medication is administered during a cancer treatment. Nephrotoxicity is often observed when platinum complex comprising medications are administered in high doses and for a prolong period. Such therapeutic regimes are usually necessary for cancer treatment to possibly remove the cancer cells entirely from the body. In the presence of DPH, which counteracts the nephrotoxic side effects off the platinum complex, higher and therefore more efficient dosages of an anti-cancer drug can be administered.

In a further preferred embodiment, the cancer is a cancer hypersensitive to a platinum complex. Although DPH inhibits the formation of Pt-adducts in cells of the inner ear and the kidney tubular cells, it does not interfere with the anti-neoplastic effects of platinum-based anti-cancer drugs. Thus, the application of DPH is particularly advantageous in combination with a medication directed to a platinum hypersensitive cancer. The increased sensitivity of the cancer to a platinum complex may be natural or induced by a drug and/or a chemotherapeutic regime.

In a preferred embodiment, this sensitivity is induced by a drug and/or a chemotherapeutic regime. Medication induced ototoxicity often prevents the use of platinum-based anti-cancer drugs. Thus, by counteracting this side effect through administering DPH, the scope of platinum-based medications, in particular platinum-based anti-cancer drugs can be significantly broadened. Besides higher and more concentrated dosage regimes the application of platinum-based cancer medications may be expanded by inducing an increased platinum sensitivity in cancer cells that are not naturally sensitive to platinum at this level.

In a preferred embodiment, the cancer is selected from the group consisting of testicular cancer, ovarian cancer, cervical cancer, bladder cancer, small and non small cell lung cancer, mammary cancer, prostate cancer, pancreatic cancer, head and neck cancer, melanoma, neuroblastoma, soft tissue cancer, medulloblastoma and osteogenic sarcoma. These types of cancers have been efficiently treated using cis-platin. However, the use of this promising medication is often limited by the occurrence of renal insufficiency due to the nephrotoxic properties of cis-platin. Efficiently counteracting these side effects by DPH, in particular in combination with increasing the platinum sensitivity of cancer cells, makes platinum-based cancer treatments available for an increased number of patients.

In a preferred embodiment, DPH is administered to the subject before or concurrently with the medication. For preventing nephrotoxicity, it is preferred to administer DPH shortly before the nephrotoxic medication such that DPH is already distributed throughout the patient's body, when the platinum complex is given. Alternatively, DPH may be applied together with the medication such that both compounds distribute simultaneously. Thus, the application of DPH may easily be integrated into cancer treatments which usually comprise carefully planned therapeutic regimes. For relieving nephrotoxicity of a medication, DPH may be applied after the medication has already been administered.

In a preferred embodiment, DPH is administered in a dosage, which correlates with the dosage of the medication. The dosage of DPH may be adapted to the dosage of the nephrotoxicity inducing platinum complex, such that the dosage of DPH can be minimized according to the dosage of the nephrotoxic medication reducing the physical strain for the patient as much as possible. On the other hand, nephrotoxicity from high doses of medications can be prevented by using correlating doses of DPH.

In a further aspect, the invention is directed to DPH for preventing and/or inhibiting structural and functional damage of proximal tubular cells of the kidney in a subject during a platinum-based cancer treatment, wherein DPH inhibits the formation of platinum adducts within the proximal tubular cells. The proximal tubules form the outermost single-layered epithelium of the kidney. They reabsorb water, minerals and nutrients from the primary urine and reintroduce them into the body's nutrient circulation. The dysfunctions of renal tubules can lead to excessive loss of water, minerals and nutrients through excretion and even to the activation of the immune system causing severe inflammation of the kidney. Therefore, it is essential to minimize nephrotoxic side effects of platinum-based medications as e.g. cis-platin. This can be achieved by co-administering DPH, which inhibits the formation Pt-adducts and thereby prevents structural and functional damages of proximal tubules due to the platinum containing medication. This is particularly advantageous, since renal dysfunction generally only becomes apparent after severe damages have already manifested. Thus, a preventive application of DPH is suitable to avoid the need of further medications to treat kidney damages as a result of the administration of platinum complexes.

In a further aspect, the invention is directed to an agent for treating nephrotoxicity induced by a platinum complex, wherein the agent interferes with a modulator of the import and/or the export of the platinum complex into/from a proximal tubular cell. The inventor found that upon administration of a platinum complex Pt-adducts specifically accumulate within the kidney in proximal tubular cells. Since these cells, in contrast to other cells of the kidney, express high levels of various different transporter molecules, the specific accumulation of Pt-adducts is believed to be due to the specific import of platinum complexes into the cells by endogenous transporter molecules. Thus, accumulation of Pt-adducts in proximal tubules may be inhibited by an agent specifically interfering with such transport molecules. The agent can decrease the expression of a transporter within the proximal tubules or inhibit its activity, e.g. by competing with the platinum complex for the binding site of the transporter. Accordingly, a method for treating nephrotoxicity in a subject induced by a medication comprising a platinum complex is disclosed comprising administering to the subject an agent which reduces the import of the platinum complex into the proximal tubular cells of the kidney.

In a preferred embodiment, the agent inhibits an endogenous transporter of the platinum complex, wherein the transporter imports the platinum complex into the cell. In an alternative embodiment, the agent promotes an endogenous transporter of the platinum complex, wherein the transporter exports the platinum complex from the cell. Besides marginal cells and outer hair cells, the inventor also found specific kidney cells, namely proximal tubular cells, to accumulate Pt-adducts upon administration of cis-platin. Similar to the situation observed in the inner ear, cells surrounding the platinum accumulating proximal tubules did not show elevated Pt-adduct levels. Since the accumulation of Pt-adducts in marginal cells and outer hair cells as well as proximal tubules always occurred simultaneously, it is suggestive that the accumulation in all cell types depends on similar transport mechanisms and can be inhibited by interfering with these mechanisms.

In a further aspect, the invention is directed to Diphenylhydramine (DPH) for treating ototoxicity, nephrotoxicity and/or neurotoxicity in a subject, wherein the ototoxicity and/or nephrotoxicity is induced by a medication comprising a platinum complex. Upon administrating a platinum complex comprising medication as e.g. cis-platin, platinum complexes are taken up by fast dividing cancer cells where they form Pt-adducts leading to structural and functional damages, finally causing the death of the cancer cell. However, the inventor could show that besides cancer cells, the platinum complexes are also taken up by specific cells of the inner ear and the kidney where they lead to the accumulation of specifically high levels of Pt-adducts. Furthermore, these accumulations cause the ototoxic and nephrotoxic side effects regularly observed upon administration of platinum-based medications. Finally, the inventor could show that the accumulations of Pt-adducts in cells of the inner ear and the kidney can be inhibited by the administration of DPH along side the platinum complex, while not altering its anti-neoplastic effects.

Thus, DPH provides a suitable compound for platinum induced ototoxicity and/or nephrotoxicity. Accordingly, a method is disclosed for treating ototoxicity and/or nephrotoxicity in a subject, comprising the administration of DPH, wherein the ototoxicity and/or nephrotoxicity is induced by a medication comprising a platinum complex.

In a further aspect, the invention is directed to an *in vitro* method for determining a subject's susceptibility for platinum induced ototoxicity, platinum induced nephrotoxicity and/or neurotoxicity, comprising the steps of determining the subject's genetic variant of a platinum complex transporter, and correlating the genetic variant of the platinum complex transporter to the susceptibility of platinum induced ototoxicity and/or platinum induced nephrotoxicity. The efficiency with which platinum complexes are transported into or out of a cell depends, *inter alia*, on the amount of transporter proteins present in the cell. The expression of the protein, in turn, is influenced by the gene from which it is translated. This is because, besides other factors, the DNA sequence of the gene determines the efficiency with which transcription factors bind to the DNA and interact with further co-factors. In addition, an altered genetic sequence can lead to a modified amino acid sequence of the protein, changing the proteins activity. Thus, the level of expression as well as the level of activity of a protein, e.g. a transporter molecule is influenced by the sequence of the protein's gene. Therefore, the levels of platinum complexes present in the marginal cells and outer hair cells of the inner ear as well as in the proximal tubular cells can substantially differ between patients dependent on the genetic variant of the platinum transporter, which they carry. This notion is in accordance with the fact that ototoxicity as well as nephrotoxicity does not occur in all patients in response to platinum containing medication. Furthermore, different intensities of hearing damages are observed in connection with cis-platin treatments. Thus, patients show diverse susceptibility for platinum induced ototoxicity and platinum induced nephrotoxicity. In regard to the efforts made to adapt a therapeutic regime to the patient's individual condition, it is desirable to predict whether a patient is likely to develop hearing damages and/or renal insufficiency in respond to a platinum containing medication. To determine this, the invention provides a method in which the patient's genetic variant of a platinum complex transporter is determined and correlated with the susceptibility for platinum induced ototoxicity and/or platinum induced nephrotoxicity. This may be achieved by comparing the genetic variant to a library of different genetic variants of the transporter in which the susceptibility for platinum induced ototoxicity and/or platinum induced nephrotoxicity is known for each variant. Alternatively, the amino acid sequence of the transporter may be determined from the genetic sequence providing information on whether the transporter is expected to efficiently import or export a platinum containing compound e.g. cis-platin. According to the patient's susceptibility, the platinum complex may be administered alone or in combination with an agent inhibiting the accumulation of Pt-adducts in marginal cells, outer hair cells and/or proximal tubular cells. In cases of extremely high susceptibility for developing hearing loss or renal insufficiency in response to platinum-based medication, alternative therapy regimes may be chosen. In addition, using DNA sequencing methods DNA libraries for platinum complex transporter gene can be established allowing regular screenings for genetic variants of the transporter. By identifying the patient's germ line transporter gene variants, which will be expressed in the cells of the inner ear, the kidney and the DRG, the patient's susceptibility to cis-platin side effects can possibly be determined prior to any exposure to the cis-platin or derivatives. In addition, the transporter variant expressed in the patient's tumors can be analyzed e.g. from a biopsy, allowing determining whether the gene of the transporter underwent mutations during tumor formation.

In a further aspect, the invention is directed to an *in vitro* method for screening a compound for inhibiting ototoxicity, nephrotoxicity and/or neurotoxicity induced by a platinum complex, comprising the steps of providing a test sample comprising cells derived from the organ of Corti, the stria vascularis of the inner ear and/or the proximal tubules of the kidney, incubating the test sample with the compound, adding the platinum complex to the test sample, determining the amount of platinum adducts formed by the platinum complex in the test sample, and comparing the level of platinum adducts formed in the test sample to the level of platinum adducts formed in a control sample, wherein a reduced amount of platinum adducts formed in the test sample in comparison to the control sample indicates the compound's potential to inhibit ototoxicity and/or nephrotoxicity induced by a platinum complex. Using an antibody directed against Pt-GG adducts, the inventor found that platinum complexes are not incorporated into all cells equally but accumulate to particularly high levels in few specific cell types of the inner ear, namely marginal cells and outer hair cells of the inner ear and in proximal tubules of the kidney. Due to the accumulated platinum adducts, the cells undergo structural and functional alterations that finally lead to substantial loss of function and cell death, which manifests in hearing loss and renal insufficiency. Both, ototoxicity and nephrotoxicity of platinum complex containing medications can be prevented by administering a compound inhibiting the accumulation of Pt-adducts in marginal cells and outer hair cells of the inner ear as well as proximal tubular cells of the kidney, respectively. Along these lines, the invention provides an *in vitro* method for screening such compounds. At first, a test sample is provided comprising cells from the organ of Corti, the stria vascularis of the inner ear or proximal tubules of the kidney. These cells may be provided as *in vitro* cultures, explants or stable cell lines, wherein the latter are particularly preferred since they allow for consistent and repeatable experimental conditions. Suitable cell lines can be kidney proximal tubular cell lines, as e.g. the cell line LLC-PK1, immortalized cell lines from the organ of Corti or chochlea cell lines (Kalinec *et al.*, 2003). The cells are incubated with a compound which is to be tested for its potential to inhibit ototoxicity and/or nephrotoxicity induced by a platinum complex. This compound may be a small molecule, a natural compound, an already known drug having further pharmacological properties or a newly synthesized chemical agent. In a preferred embodiment, the method is applied using high through put processes such that a variety of compounds, preferably entire substance libraries, may be screened at once. After incubating the cells with the compound, a platinum complex is added to the sample and incubated to give the cells time to take up the complex and form Pt-adducts. A compound, which reduces or prevents the uptake of the platinum complex and thus the formation of Pt-adducts is expected to have ototoxicity and/or nephrotoxicity inhibiting properties. Alternatively, the cells may be exposed to the platinum complex before they are incubated with the compound, such that the compound is tested for its ability to increase the export of platinum complexes from the cells or even dissolving preformed Pt-adducts. Such a compound is expected, too, to be suitable for treating platinum induced ototoxicity and/or nephrotoxicity. After removing the supernatant containing the compound and/or the platinum complex from the sample, the amount of Pt-adducts formed in the cells is determined, e.g. by using an antibody directed against Pt-GG adducts. To evaluate the effect of the compound on the formation of Pt-adducts, the level of Pt-adducts formed in the cells of the test sample is compared to the level of Pt-adducts formed in cells of a control sample. The control sample is treated in parallel to the test sample, however, in the absence of a compound. Optionally, a positive control sample may be included in the method treating a sample of cells in parallel to the test sample using DPH as a Pt-adduct inhibiting compound. If a test sample shows reduced levels of Pt-adducts compared to the control sample, preferably similar or even reduced levels compared to a DPH sample, the compound is expected to be suitable to prevent marginal cells, outer hair cells and proximal tubules from platinum induced damages and thus to inhibit hearing loss and renal insufficiency in response to platinum containing medication. Accordingly, a method for screening a compound for inhibiting ototoxicity, nephrotoxicity and/or neurotoxicity induced by a platinum complex is disclosed comprising the steps of administering the compound to a test animal, preferably a mouse, determining the amount of platinum adducts formed by the platinum complex in cells of the test animal and comparing the level of platinum adducts formed in the cells of the test animal to the level of platinum adducts formed in cells of a control animal, wherein a reduced amount of platinum adducts formed in the test animal in comparison to the control animal indicates the compound's potential to inhibit ototoxicity and/or nephrotoxicity induced by a platinum complex.

In a further aspect, the invention relates to a kit for screening a compound, comprising cells derived from the organ of Corti, the stria vascularis of the inner ear and/or the proximal tubules of the kidney, a platinum complex, and an antibody against platinum adducts. This kit provides a suitable tool to test single compounds as well as series of compounds for their potential to inhibit ototoxicity and/or nephrotoxicity induced by a platinum complex. The cells may be provided as frozen samples, preferably derived from stable cell lines. The platinum complex is preferably selected from the group consisting of cis-diaminedichloro-platinum(II) (cis-platin), cis-platin analogs, amine platinum complexes, diamine platinum complexes, trans-platinum complexes and multinuclear platinum complexes Platinum(IV) coordination complexes. The antibody is directed against Pt-adducts, preferably it is an anti-Pt-GG antibody as e.g. the monoclonal antibody R-C18 (Liedert *et al.*, 2006). The antibody may be directly labeled or may be detectable using a secondary antibody having a label, e.g. a fluorochrome. Using the kit of the invention, a given compound is tested for its ability to decrease the formation of Pt-adducts in cells of the inner ear and/or proximal tubules of the kidney. For this, the cells are incubated with the compound in question before or after they are treated with the platinum complex. The level of Pt-adducts formed in the presence or in the absence of the compound are determined using the antibody and compared to each other. A reduced formation of Pt-adducts in the presence of the compound indicates the compounds ability to prevent platinum induced ototoxicity and/or nephrotoxicity by inhibiting the formation of Pt-adducts.

In a further aspect, the invention is directed to a composition for treating cancer and concurrently inhibiting ototoxicity and/or nephrotoxicity, wherein the composition comprises cis-platin and DPH. Cis-platin is one of the most frequently used anti-cancer drugs. Its application is, however, limited due to severe side effects, in particular to loss of hearing and renal insufficiency. The inventor found that both the ototoxic and the nephrotoxic properties of cis-platin are caused by prominent accumulations of Pt-adducts in specific cells of the inner ear and the kidney. The inventor further found that this accumulation is inhibited when cis-platin is administered together with DPH. Without interfering with the anti-neoplastic effect of cis-platin, DPH prevents the accumulation of Pt-adducts in the inner ear and the kidney such that a composition comprising both compounds is particularly suited for treating cancer while minimizing the ototoxic and nephrotoxic side effects of cis-platin. Accordingly, a method for treating cancer and inhibiting ototoxicity and nephrotoxicity in a subject is disclosed, comprising administering to the subject a composition comprising cis-platin and DPH.

In a preferred embodiment, the composition comprises a further anti-cancer drug, selected from the group consisting of a cytotoxic agent, a cytostatic agent, a therapeutic antibody, a small synthetic molecule, a small naturally occurring molecule, a small coding RNA, a small non-coding RNA and a cytokine. The non-coding RNA may be siRNA, microRNA or shRNA. For sufficient cancer treatment it is essential to kill as many cancer cells as possible in the shortest time possible to prevent them from repopulating. Therefore, combinatorial approaches using either multiple compounds of a single class of anti-cancer drugs or several compounds belonging to different classes of anti-cancer drugs have been developed. Including DPH into the composition attenuates the ototoxic and nephrotoxic side effects of cis-platin reducing the pharmacological stress of the patient and allowing the application of a further anti-cancer drug which might not be possible in cases where patients show particularly strong ototoxic or nephrotoxic reactions to cis-platin. For supporting the anti-neoplastic effect of cis-platin, the drug is preferably combined with etoposide, paclitaxel, doxorubicine, 5-fluorouracil, gemcitabine, vinblastine or bleomycin. Alternatively, the composition may comprise therapeutic antibodies or small molecule weight inhibitors, preferably kinase or PARP inhibitors in addition to cis-platin.

In a preferred embodiment, the composition is administered during a radiation therapy. Combinations of chemo- and radio-therapeutic approaches are applied increasingly often, since they efficiently eliminate cancer cells reducing the risk of their repopulation and thus the reappearance of the cancer. However, they are particularly exhausting for the patient and further impair the patient's health conditions. Using a composition comprising cis-platin and DPH together with a radiation therapy reduces the drug induced side effects, namely the ototoxicity and the nephrotoxicity of cis-platin, thereby reducing the patient's overall burden and improving the recovery potential.

In a further aspect, the invention is directed to a composition for treating infection and concurrently inhibiting ototoxicity, nephrotoxicity and/or neurotoxicity, wherein the composition comprises an antibiotic and DPH. Several antibiotics as for example capreomycin, chloramphenicol and gentamycin are known to have ototoxic as well as nephrotoxic side effects. The inventor found that DPH efficiently inhibits drug induced ototoxicity and nephrotoxicity. Thus, when using an antibiotic known to cause hearing damages and renal insufficiency, the medication is administered in a composition together with DPH counteracting the side effects of the antibiotic. Accordingly, a method for treating an infection and inhibiting ototoxicity and/or nephrotoxicity in a subject is disclosed, comprising administering to the subject a composition comprising an antibiotic and DPH.

In a further aspect, the invention is directed to a composition for treating a kidney dysfunction and concurrently inhibiting ototoxicity, nephrotoxicity and/or neurotoxicity, wherein the composition comprises a diuretic and DPH. Some diuretics as e.g. furosemide, bumecanide and ethacrynic acid are known to show ototoxic side effects. Using a composition according to the invention, the kidney disease is treated by diuretics while the undesired side effect is simultaneously counteracted by DPH. Accordingly, a method for treating a kidney dysfunction and inhibiting ototoxicity and/or nephrotoxicity in a subject is disclosed, comprising administering to the subject a composition comprises a diuretic and DPH.

In a further aspect, the invention is directed to an agent increasing a cell's sensitivity to a platinum complex, wherein the agent induces and/or enhances the expression and/or activity of a platinum complex transporter in the cell. Despite their non-proliferative state, marginal cells and outer hair cells of the inner ear as well as proximal tubular cells were shown to take up high amounts of platinum complexes that intercalate in the cells DNA, forming Pt-adducts. This high uptake of platinum complexes is most probably due to the active import by endogenous transporter molecules, presumably specifically common to marginal cells and outer hair cells of the inner ear as well as proximal tubular cells. The artificial introduction of such a transporter molecule into a cell may therefore increase the cell's sensitivity to a platinum complex. Such a modified cell may be suitable for further studying the cellular and molecular mechanisms underlying the anti-neoplastic effect as well as undesired side effects of platinum complexes as e.g. cis-platin. Moreover, the artificial introduction of a platinum complex transporter provides therapeutic applications by increasing the sensitivity of the cancer cells. The transporter can be introduced into the cancer cells by genetic manipulation or as a preformed protein delivered using e.g. a viral envelop. By increasing the cancer cells' sensitivity to cis-platin the efficiency of cis-platin based cancer therapies can by improved. Accordingly, a method for increasing a cell's sensitivity to a platinum complex is disclosed comprising administering an agent to the cell, wherein the agent induces and/or enhances the expression and/or activity of a platinum complex transporter in the cell.

In a further aspect, the invention is directed to DPH for treating neurotoxicity in a subject, wherein the neurotoxicity is induced by a medication, preferably a medication comprising a platinum complex. Besides ototoxicity and nephrotoxicity cis-platin induces damages of neurons, in particular of dorsal root ganglion (DRG) neurons and their satellite cells, leading to functional loss in the peripheral nervous system. This has been related to increased accumulations of Pt-adducts found upon administration of cis-platin in DRG neurons causing DNA damage and cell death. However, administrating DPH to mice treated with cis-platin significantly reduced the formation of PT-adducts in DRG neurons. Thus DPH exerts a neuroprotective effect on DRG cells and is thus suitable for treating neurotoxicity, preferably treating neurotoxicity and ototoxicity and/or nephrotoxicity.

### Examples

### 1. Material and Methods

### Breeding and treatment of mice

All animals were kept under specific pathogen free conditions at 12 hours light/dark cycle with free access to water and standard laboratory mouse food and water *ad libitum*. All experiments have been approved by the state animal welfare board. Mice were weighed and checked for signs of drug toxicity before each application of cis-platin. For experiments with wild type mice male C57BL/6J mice (or BALB/c mice for electron microscopy studies) of 12-14 weeks were used. Except indicated otherwise, drugs were administrated by i.p. injections and treatment regimens were as follows: 10 mg/kg of cis-platin (acute, single treatment) or 5 mg/kg of cis-platin (plus 500 µl of saline solution) every second day for 8 days (cumulative dose: 20 mg/kg of cis-platin; chronic treatment). In the case of inhibitor treatment, the inhibitors were administered 1 hour before cis-platin treatment. In following studies after first check, diphenhydramine (DPH) was administered 30 minutes before cis-platin. For experiments with mice bearing lung tumors, 8 weeks old mice were infected with AdCre (University of Iowa) by nasal inhalation (Oliver *et al.*, 2010) and further kept for 12-16 weeks to allow the development of lung tumors. Treatment of mice was done by i.p. injections in three schedules. Acute treatment: 10 mg/kg of cis-platin with or without inhibitor DPH 57 mg/kg. Chronic treatment: 5 mg/kg of cis-platin with or without DPH plus saline buffer. Long-term treatment to study tumor regression: mice were treated once per week with 7 mg/kg of cis-platin, 4 times a month. Long term treatment to study drug response of tumors: 4 consecutive treatments once per week (7 mg/kg of cis-platin). For experiments with XPA-/- mice, 10-16 week old males were used and treated with cis-platin twice (1 mg/kg on the first day and 2.5 mg/kg after one week). After each cis-platin treatment an i.p. injection of saline (500 µl) was given to prevent kidney toxicity.

### Tissue preparation and frozen tissue section

The sacrificed animals were immediately dissected. Kidneys and lungs were submerged in embedding medium (Tissue-Tek O.C.T. Compound) and frozen in liquid nitrogen. Cochleae were dissected and fixed by intralabyrinthine perfusion with Carnoy's solution (3 hours; for DNA adduct staining) or with formaldehyde (4 % in PBS; 1 hour; for OCT protein staining). Cochleae were decalcified in EDTA solution (10 % w/v in PBS) for 3 days and then placed in sucrose solutions (5 % in PBS for 12 hours and 15 % in PBS for 5 hours). After that samples were imbedded in Tissue-Tek medium, frozen and cryosections of 10 µm cut.

### Visualization and measurement of Pt-adducts - immunocytological assay

### Solutions:

*Alkali solution*: 60 % 70 mM NaOH / 140 mM NaCl, 40 % methanol
*Proteinase K-Buffer*: 20 mM Tris 2 mM CaCl₂, pH 7.5
*Glycin*/*PBS*: 0.2 % Glycin in PBS
*PBST*: 0.05 % Tween 20 in PBS

Frozen tissue sections of the inner ear or kidneys on ImmunoSelect slides were fixed in methanol at -20°C for at least 2 hours or overnight and then rehydrated for 10 minutes in PBS, at room temperature. To provide better antibody penetration in the tissue structures the samples underwent alkaline permeabilisation of cytoplasmic and nuclear membranes for 5 minutes at 0°C, followed by washing in PBS at room temperature for 5 minutes. The proteolytic cleavage of cytoplasmic and nuclear proteins was performed in two steps. At first, a pre-warmed pepsin solution (60 µg /ml for cell culture or 100 µg/ml for tissue sections in PBS/HCl) was applied onto the tissue sections and incubated for 10 minutes at 37°C in a humidified chamber. The incubation with pepsin was followed by washing in PBS for 10 minutes at room temperature. In a second step tissue sections were incubated with pre-warmed proteinase K solution (40 µg/ml for cell cultures or 50-100 µg /ml for tissue sections in proteinase K buffer) for 10 minutes at 37°C in a humidified chamber. Subsequently the sections were washed in 0.2 % glycine in PBS for 10 minutes at room temperature. Non-specific binding of the antibodies was inhibited by incubation with a blocking solution of 1 % casein in PBS for 30 minutes at room temperature. The samples were then incubated with a monoclonal antibody (R-C18, Liedert *et al.,* 2006) specific for Pt-GG adducts in DNA (0.1 µg/ml in 0.1 % BSA/PBS) over night at 4°C in the humidified chamber. Subsequently, the slides were washed in 0.05 % Tween 20 in PBS for 5 minutes at room temperature and then in PBS for 5 minutes. The incubation with secondary Cy3-labeled rabbit anti-(rat Ig) antibodies, was performed (0.2 µg/ml in 0.1 % BSA/PBS) for 60 minutes at 37°C in the humidified chamber, followed by washing in 0.05 % Tween 20 in PBS for 5 minutes at room temperature and then in PBS for 5 minutes. The nuclear DNA was counterstained with DAPI (1 µg/ml in PBS) for 30 minutes at room temperature. The slides were then washed in PBS. Finally, the samples were embedded with a densification compound "VECTASHIELD". The quantification of immuno- and DNA-fluorescence was performed by means of a microscope-coupled digital image analysis system ACAS 6.0 Cytometry Analysis System. Adduct levels in the nuclear DNA of individual cells were calculated by normalizing antibody-derived fluorescence signals to the corresponding DNA content of the same nucleus, and were expressed as arbitrary fluorescence units (AFUs). Data were assigned to specific cell types as determined by histochemistry.

### Electrophysiological examination of the hearing capability of mice

Auditory brainstem response or brainstem auditory evoked response (BAER) is an electrical signal evoked from the brainstem by the presentation of a sound such as a click. BAER is a clinical screening test to monitor for hearing loss or deafness assessing the functions of the ears, cranial nerves, and various brain functions of the lower part of the auditory system. The cochlea acts as a mechanical demodulator in the hearing process and is responsible for converting sound vibrations into nerve impulses. The stimulation of associated nerve endings and conduction signal through afferent nerves via the cochlear ganglia to the cochlear nucleus, then to the superior olivary complex, then to the inferior colliculus, then to the medial geniculate body and finally to the auditory cortex. All electrophysiological examinations were carried out under general anesthesia (xylazine, 20 mg/kg, plus ketamine, 100 mg/kg; i.p.). To minimize effects of body temperature on the evaluation process, animals were placed to a heated (37°C) thermal blanket. Subdermal needle electrodes were inserted at vertex (active electrode), ventrolateral of left pinna (reference electrode) and ventrolateral of right pinna (ground electrode). For acoustic stimulus presentation, auditory brainstem response recording and data management a modified clinical potential system (NeuroScreen Plus, Toennies, Germany) was used. Repetitive clicks (substantial energy: 1-3 kHz; duration: 10 ms, rate: 39 /s) were delivered through plastic tubes (diameter: 3 mm) to both ear canals (EAR-Auditory Systems, Indianapolis). BAER thresholds were determined by clicks beginning at 90 dB sound pressure level with decreasing intensity (10 dB-steps) until the waves have lost reproducible morphology. The BAER thresholds were determined separately for each ear and by repetitive measurements. Following BAER, mice were sacrificed by cervical dislocation and both cochleae were removed for further analysis.

### 2. Results

### 2.1 Establishing a mouse model for cis-platin induced ototoxicity

The cellular and molecular mechanisms underlying the organ specific toxicity of cis-platin in the inner ear and in the kidney have been studied predominantly in mammalian cell culture systems employing immortalized physiological cells such as proximal tubule cell lines. The majority of these studies were unable to explain the particular sensitivity of the target tissues to cis-platin because the cell systems resembled neither the complex pharmacokinetic situation within the affected organs nor the particular response of terminally differentiated, non dividing cells to the drug. It is widely accepted that the cells of the kidney which are damaged predominantly during cis-platin treatment, and, are responsible for the nephrotoxicity and general renal dysfunction, are the epithelium cells of the proximal tubules (Arany and Safirstein, 2003). This had been evidenced by morphological studies in patients and animals treated with cis-platin as well as by comparisons of various kidney cell lines developed from different cell types. The inner ear damage after cis-platin treatment was detected mainly by electron microscopic analysis but also by morphological studies of the cochlea (Klis *et al.*, 2002; Cardinaal *et al.*, 2004;). The main conclusions from the aforementioned studies were that: (1) cis-platin-treated animals lost their cochlear hair cells, (2) the damage of the cochlea by cis-platin begins from the basal turn, (3) cis-platin causes degeneration of the stria vascularis and the spiral ganglion neurons. It has to be mentioned that almost all animal studies on the inner ear have been done so far with rats or guinea pigs, as cochleae of these animals are large enough to allow easy handling. The conclusions drawn from these studies neither provided clear evidence, how to design preventive treatment strategies, nor did they help to identify the primary molecular targets of cis-platin derived ototoxicity and discriminate them from secondary effects. As no suitable transgenic rat or guinea pig strains for more detailed studies were available the inventor first established a mouse model to analyze the parameters targeting the toxicity of cis-platin to specific structures within the inner ear and the kidney.

### Cochlea and kidney harbor specific target cells for cis-platin

Male C57BL/6J mice (age 12 weeks) were injected i.p. with a single dose of cis-platin (10 mg/kg). 24 hours later, the inner ears and kidneys were dissected, kidneys were frozen immediately and cochleae were fixed and decalcified. Cryosections (10 µm) prepared from the tissues were placed onto specific adhesion slides. Slides were then immunostained for the visualization of platinum-guanine-guanine (Pt-GG) intrastrand adducts in the nuclei of individual cells by using the adduct-specific monoclonal antibody R-C18 in an immunocytological assay (ICA) (Liedert *et al.,* 2006). The tissue sections were then stained with a secondary Cy3-labeled goat-anti-rat antibody (Cy3), counterstained with DAPI for nuclear DNA, and analyzed in the fluorescence microscope (Figure 1). Serial sections from the same tissues were stained for morphological orientation with hematoxyline and eosin (H&E). This analysis revealed a drastically unequal distribution of the nuclear DNA platination among cochlear cells. Two particular areas, parts of the organ of Corti and cells of the stria vascularis depicted extremely high levels of Pt-GG adducts whereas other structures were stained to a far lower degree (Figure 1 A). Micrographs of the organ of Corti at higher magnification (60x) clearly demonstrated high levels of DNA platination exclusively in the outer hair cells (OHC) but not in inner hair cells (IHC) or other cell types of organ of Corti (Figure 2 A). More detailed analysis of the stria vascularis revealed a similar situation. High adduct levels were visible in this structure only in the outer cell layer representing the marginal cells, but not in the neighboring intermediate or basal cells (Figure 2 B). A similar observation was made for kidney tissues, where the DNA of proximal tubular cells carried very high adduct burdens, whereas other types of cells depicted much lower levels of DNA platination (Figure 1 B).

### Quantification of Pt-GG adducts in the nuclei of cochlear cells

For the numerical determination of the DNA platination levels in the nuclei of individual cells, a Zeiss Axioplan fluorescence microscope coupled to a quantitative multichannel digital image analysis system (ACAS 2) was applied. This system automatically localizes single nucleus areas due to the DAPI-derived signals from DNA and integrates the intensities of the antibody-derived Cy3 signals for the same pixels (Liedert *et al.,* 2006). Relative adduct levels in the nuclear DNA of individual cells were calculated by normalizing antibody-derived fluorescence signals to the corresponding DNA content of the same cell (in order to correct for a possible DNA loss) and were expressed as arbitrary fluorescence units (AFUs). The AFU values were used as quantitative parameters and mean values (± SD) from 50 or 100 cells from the organ of Corti or the stria vascularis, respectively, were calculated to describe the relative adduct levels in particular cell types. Mice were injected i.p. with 10 mg/kg of cis-platin and sacrificed after 24 h or 48 h. Cryosections of cochlea and kidney tissues were stained with anti- Pt-GG antibody and signals were measured from single nuclei of different types of cells. When analyzing the immunostained cochlea sections, four types of cells were measured separately: (1) outer hair cells, (2) surrounding cells (supporting cells of the organ of Corti), (3) marginal cells of the stria vascularis and (4) adjacent cells (intermediate and basal cells of the stria vascularis) (Figure 3). In the kidney, proximal tubule cells, distal tubule cells and cells of the glomerulus were measured in the same way. The platination level in distal tubular cells was twice as high as that in glomerulus cells (2.61 AFUs vs. 0.97 AFUs) whereas the proximal tubular cells showed the highest level of platination (7-fold higher than distal tubular cells: (18.97 AFUs vs. 2.61 AFUs) (Figure 3 A). The levels of Pt-GG adducts in the highly platinated cells and directly neighboring cells of the cochlea were dramatically different being 11-fold higher in OHC vs. adjacent cells in the organ of Corti (13.26 AFUs vs. 1.19 AFUs), and 5-fold higher in the marginal cells as compared to the other cell types in the stria vascularis (9,85 AFUs vs. 2.08 AFUs) (Figure 3 B).

### Different adduct levels in basal, middle and apical turns of the cochlea

Previously differences in the extent of morphological damage between basal and apical turns of the cochlea in the stria vascularis marginal cells of cis-platin-treated rats were described (Cardinaal *et al.,* 2004). To test whether such observations are correlated to differences in DNA platination, analyses were performed with cryosections from all four turns of the cochlea from a mouse 24 h after cis-platin treatment. The quantitative measurement supported the structural observations by showing a decrease in marginal cell adduct levels from the basal to the apical turn (Figure 4). A similar trend, although at nearly 10-fold lower levels, was also observed for the non-marginal cells of the stria vascularis. This finding corresponds to the clinical observations that cis-platin-induced hearing loss begins with high frequencies, which are recorded predominantly in the basal turn of the cochlea (Sluyter *et al.,* 2003).

### 2.2 Pt-adducts in hair cells, marginal cells and proximal tubule cells

The dramatic accumulation of platinum adducts in particular cell types in the cochlea and the kidney of cis-platin-treated mice raised the question of the mechanisms underlying that observation. Basically, all the functions discussed for drug susceptibility of tumor cells (i.e. accelerated cellular uptake or decreased export of the drug, abrogation of the intracellular deactivation, or the incapability to repair adducts once formed in the DNA) might also be involved in physiological cells, and could all lead to the observed differential accumulation of platinum adducts.

### Formation and repair of Pt-GG adducts in the DNA of different inner ear cells

It had been shown that Pt-GG intrastrand cross links can be removed efficiently from the nuclear DNA of cells in various mouse tissues by the nucleotide excision repair pathway (Dzagnidze *et al.*, 2007). Therefore, it was first analyzed whether a deficiency in DNA repair might be responsible for the high adduct accumulation in OHCs or marginal cells of the cochlea. C57BL/6 mice were treated with a single dose of cis-platin (12.5 mg/kg) and three animals were sacrificed at each of the different time points (2, 16, 48 and 72 h) after injection. Adduct levels were measured by ICA in three cell types of the cochlea, namely outer hair cells, marginal cells and cells of Reissner's membrane (RM) (Figure 5). The latter only showed weak immunofluorescence signals at all time points after cis-platin treatments, and were chosen as an example for low adducted cochlear cells. The OHCs and the marginal cells depicted high levels of adduct formation after cis-platin treatment and were designated as target cells. DNA platination was detectible in all three cell types already 2 hours after cis-platin administration. The Pt-GG levels peaked after 16 hours in OHCs and after 48 hours in marginal. They returned to comparatively low values in all three cell types 72 hours after treatment. As expected from the previous experiments, the adduct levels of the RM cells were only about 10 % of those of the two other cell types. The repair kinetics, however, clearly demonstrated that the excessive DNA platination in both OHC and marginal cells is not caused by their inability to repair such lesions. This observation strongly implied that factors other than repair deficiency are responsible for the exorbitant DNA damage in the two target cell types. As mentioned above, proximal tubule cells of the kidney have physiological similarities with cochlear hair and marginal cells, namely their capacity of intensive unidirectional ion transport.

### Localization of organic cation transporters in the cochlea

To identify potential molecules that may cause an aberrant active uptake of cis-platin, it was analyzed whether some membrane transporters are expressed in the highly adducted cells in the kidney and in the inner ear, but not in the surrounding cells. Along these lines expression of the organic cation transporter 2 (OCT 2) and 3 (OCT 3) were examined in the murine inner ear. Eight micron thick tissue sections were prepared and immunostained for OCT 2 and OCT 3 using specific antibodies (Alpha Diagnostic International, BioTrend). OCT 2 and OCT 3 were positively stained in intermediate and basal cells of the stria vascularis, in supporting cells of the organ of Corti (cells surrounding the outer hair cells) and even in inner hair cells (Figure 6). However, no signals were visible in the marginal cells and the outer hair cells.

### 2.3 Correlation of levels of platinum-DNA adducts and hearing loss

It is generally accepted that the anti-neoplastic effect of platinum anti-cancer drugs is mediated via the formation of adducts in the nuclear DNA of tumor cells and the subsequent induction of apoptotic pathways. For drug-induced damage and functional loss in physiological cells, however, this route of action is by far less well established and a matter of constant controversial discussion in the literature. To further investigate the accumulation of DNA platination products that caused ototoxicity, two isogenic mouse strains (C57BL/6 NER wild type and XPA-/-) were compared which differ solely in their ability to repair cis-platin intrastrand adducts in their genomic DNA. While the wild type mice have a fully functional nucleotide excision repair (NER) system, a homozygous deficiency for the xeroderma pigmentosum (XP) A protein, a major component of the NER pathway, has been shown to reduce (but not to completely abrogate) the repair capacity for Pt-GG intrastrand cross links in the DNA of various tissues (Liedert *et al.,* 2006; Dzagnidze *et al.*, 2007).

### Increased cis-platin ototoxicity in XPA knockout mice

As a functional test for the hearing capability of mice the method of brainstem auditory evoked response (BAER) was employed. XPA -/- mice and their wild type littermates were treated with cis-platin at the particular low cumulative dose of 3.5 mg/kg within two weeks (1^{st} week: 1 mg/kg; 2nd week: 2.5 mg/kg). Control groups of both strains received PBS injections instead. The hearing capability of animals in all four experimental groups was determined by BAER measurement 48 hours after the last treatment. The treated and the control groups of the repair-proficient mice did not exhibit any sign of cis-platin ototoxicity (Figure 7 A). The control group of the repair-deficient XPA-/- mice exhibited the same hearing threshold, indicating that the status of the NER system itself did not result in any hearing problems. In contrast, cis-platin treated NER-deficient XPA-/- mice exhibited a reduction of their hearing capability of nearly 50 %, indicating that unrepaired DNA adducts indeed represent the initial molecular trigger for the functional hearing loss.

### Higher accumulation of DNA adducts in cochlear cells of XPA-deficient mice

To further substantiate this indication, the mice of all four experimental groups were sacrificed immediately after the BAER test and cochleae were taken for adduct specific immunostaining with anti-Pt-GG monoclonal antibodies. The ICA revealed about 2.7-fold higher levels of DNA damage in the marginal cells of cis-platin-treated XPA-1- mice compared to their wild type littermates (Figure 7 B). Thus, the platinum adduct levels correlated well with the functional loss in the hearing system.

### 2.4 A strategy for the prevention of cis-platin induced hearing loss

Having demonstrated the role of unrepaired cis-platin-DNA damage in hearing loss and its excessive formation rates in target cells of the affected tissues, strategies for preventing the severe side effects of cis-platin treatment, such as ototoxicity, were investigated. For examining the possibility to interfere with the formation of Pt-adducts upon cis-platin treatment, the inventor performed an *in vivo* screening for identifying pharmaceutical inhibitors of platinum induced ototoxicity.

### Screening for cis-platin uptake inhibitors

A group of small molecules was chosen as candidate compounds for an *in vivo* screening for cis-platin uptake inhibitors: quinine, butylscopolamine, diphenhydramine and ranitidine. The mice were separated into two groups for pretreatment with low or high doses of the test compounds. One hour before the treatment with cis-platin (10 mg/kg) the high dose group received i.p. injections of either quinine (100 mg/kg), ranitidine (100 mg/kg), diphenhydramine (60 mg/kg) or butylscopolamine (20 mg/kg). The low dose group received half the dose of the high dose group (50, 50, 30 or 10 mg/kg, respectively). As controls, two mice were treated with PBS instead of inhibitor prior to cis-platin (positive control) and two mice were treated with PBS only (negative control). All mice were sacrificed 24 hours after cis-platin treatment. Cochleae and kidneys were dissected for immunostaining and ICA analyses of Pt-GG adduct levels in specific cell types (Figure 8). In tissue sections from mice pretreated with quinine or butylscopolamine no inhibition of DNA platination was observed, independent of the dosing, in either of the three target cell types (renal tubulus epithelium cells (Figure 8 A), cochlear marginal cells (Figure 8B) and outer hair cells (Figure 8 C)). In contrast, high dose treatment with ranitidine had a slightly protective effect (15 - 20 % reduction of adduct levels) in all three cell types, whereas the lower dose provided no protection. Treating the mice with diphenhydramine provided a dose dependent and by far the strongest shielding from DNA damage, ranging from 75 % in proximal tubule cells over 70 % in cochlear marginal cells to about 50 % in outer hair cells at the high dose of 60 mg/kg. No significant inhibition by any of the four inhibitors was observed in the low-adduct "non-target" cells of Reissner's membrane of the cochlea (Figure 8 D).

### Dose-dependent reduction of DNA platination by DPH

In the first set of experiments, the doses of DPH to reduce the adduct formation in the target cells corresponded to 50 % and 25 % of the LD50 dose for mice. Therefore, it was tested next whether the inhibitory effect of DPH is dose-dependent and whether it is possible to reduce the dose while maintaining the preventive activity on adduct formation. For this, mice were treated by i.p. injections with DPH at different doses between 0 and 57 mg/kg (two mice per group) 30 minutes prior to a single dose of cis-platin (10 mg/kg). The ICA for Pt-GG adducts in kidney and cochlear cells revealed a dose-dependent inhibition of the adduct accumulation in proximal tubule cells and in marginal cells (Figure 9). No significant alterations were observed in the low-platinated distal tubule cells of the kidney and in the basal and intermediate cells of the stria vascularis. This shows that the inhibitor has a specific effect on proximal tubule cells and marginal cells but has no effect on the surrounding cells (such as distal tubule cells; Figure 9 B).

### Inhibition of the accumulation of Pt-adducts in dorsal root ganglion neurons

It had been previously observed that cis-platin treatment can induce neurotoxicity, in particular cell damages in dorsal root ganglion (DRG) neurons. The inventor now found that similar to cells of the inner ear and the kidney, DRG neurons as well as their satellite cells accumulate high levels of Pt-adducts but could be inhibited by administering DPH together with cis-platin (Figure 9 C).

### Effect of DPH on the repair capacity for cis-platin-DNA adducts

As shown above, the treatment with DPH prior to cis-platin injections lead to lower levels of DNA adduct accumulation in the target cells. This could be caused by reduced intracellular drug concentrations or, alternatively, by augmented removal of lesions from the genomic DNA of the target cells. To elucidate a possible interaction of DPH with the DNA repair machinery, the inventor functionally analyzed adduct kinetics. Mice were treated with DPH (57 mg/kg) and 30 minutes later with a single dose of cis-platin (12.5 mg/kg). Three mice per time point were sacrificed at 2, 16, 48 and 72 hours after cis-platin treatment and cochleae were analyzed for Pt-GG adduct levels by ICA (Figure 10). The kinetics revealed drastically decreased DNA platination in marginal and outer ear cells in DPH pre-treated animals as compared to the corresponding cis-platin alone group at all time points, but no obvious differences in their capability to repair the damage.

### 2.5 Inhibition of hearing loss by co-administration of DPH and cis-platin

In the clinical setting, the ototoxic effect of cis-platin chemotherapy is usually observed after several treatment cycles. To mimic this situation, animals were treated every second day with comparably moderate doses of cis-platin (5 mg/kg). A subgroup of animals was additionally injected with DPH (57 mg/kg) 30 minutes before each dose of cis-platin. Both groups received additional i.p. injections with saline solution (500 µl) in order to protect the mice from kidney injury. As controls, two further groups of animals received either only the inhibitor (DPH group) or saline solution (negative control). At different cumulative doses of cis-platin all mice were measured for their functional hearing capability using the BAER test. Like in the clinical BAER tests for humans, the hearing threshold was determined as the disappearance of wave V. This value was converted into percentage of hearing capability as compared to the untreated controls (dB to %). The test was done for all animals in this experiment also prior to treatment showing that they had a normal hearing capability. The mean value of the untreated mice was set as 100 %. After reaching a cumulated cis-platin dose of 20 mg/kg, the BAER test was performed 24 h after the last treatment. Mice that received only cis-platin showed a reduction of their hearing capability to about 50 % of the controls (Figure 11). In contrast, the same dose of cis-platin, but in combination with DPH, induced a hearing loss of only 20 % (Figure 11). Mice of the control and the DPH alone groups did not show any difference in their hearing ability throughout the experiment. Another group of mice that received cis-platin in cumulated doses of 40 mg/kg plus DPH still maintained a mean hearing capability of about 70 % of the untreated controls (Figure 11). Importantly, all animals from the corresponding cis-platin-alone group displayed severe signs of acute toxicity before reaching that dose and had to be euthanized.

In all treatment groups of this experiment the mice were sacrificed immediately after the final BAER test and cochleae and kidneys were resected for the measurement of DNA platination by ICA analysis. A comparison of adduct levels in the different treatment groups was performed to find out whether repetitive low dose application of cis-platin induced the same pattern of DNA platination in both tissues as the acute high dose application, and whether the reduced hearing loss in the cis-platin plus inhibitor group was associated with a lower adduct accumulation in the critical target cells under these conditions. Massive adduct accumulation was found in the target cells of the two investigated organs, kidney and inner ear, from mice of the cis-platin alone group. In contrast, by far less adducts were found in the pertinent cells of the cis-platin plus DPH treatment group. In the kidney all cells of the cortex depicted similarly low adduct levels with no protruding proximal tubules visible (Figure 12). Similarly, in the marginal and hair cells of the cochlea much lower adduct accumulation was observed in animals co-treated with DPH as compared to the cis-platin alone group (Figure 12). This protective effect was observed in all turns of the cochlea and was more pronounced in the marginal than in the outer hair cells. The adduct levels in other cell types of the inner ear remained unchanged by DPH.

Taken together, the results of this experiment strongly support the notion that the excessive DNA damage in cochlear cells is the key trigger for the cis-platin-induced hearing loss and that an inhibitor-mediated reduction of that damage is a successful strategy to significantly prevent the functional loss. Noteworthy, the general health status of the mice in the cis-platin plus inhibitor group was better and weight loss was less severe than in the cis-platin alone group. A prolonged treatment of mice with DPH alone up to eight applications had no obvious side effects. Since the protective effect of DPH for the cochlea may be mediated by blocking an ion transporter being relevant for the physiological function of the target cells, an additional experiment was done to verify whether a treatment with DPH alone alters the hearing capability of the mice. For this, five animals measured for their basal auditory sensitivity with the BAER test were treated 24 h later with a single dose of DPH (57 mg/kg), and were re-examined one hour later again by the BAER test. All mice depicted a slight reduction in their hearing capability from 100% down to 70%. When the BAER test was repeated 24 hours later all mice had regained their normal hearing sensitivity (graphs not shown). This observation further supports the notion that DPH temporally blocks ion transporters in the inner ear and by that this prevents cis-platin accumulation in the cochlear cells.

### 2.6 DPH does not affect the therapeutic efficacy of cos-platen

The pronounced protective effect of DPH on both, DNA platination and functional loss in physiological target cells, makes it an interesting candidate for co-application during cis-platin-based cancer therapy to reduce side effects. An important factor that had to be controlled in this respect is whether DPH not only reduces the severity of the side effects but also diminishes the efficacy of cis-platin-based cancer chemotherapy.

### Co-application of cis-platin and DPH to cancer cell lines

Several human cancer cell lines established from tumors, which are usually treated with cis-platin (lung, bladder, testis) were treated with cis-platin alone or in combination with DPH. DPH showed no inhibitory effect, neither on the formation of DNA adducts (as measured by ICA) nor on the induction of apoptosis (as measured by Annexin V staining and FACS analysis).

### A mouse model for human lung cancer

Oncogenic mutations in the gene encoding the small GTPase *K-ras* are among the most frequently detected alterations in human lung cancer and approximately 30 % of all cases of human non-small cell lung cancer (NSCLC) carry such an activated *K-ras* allele with mutations predominantly located in codons 12, 13 or 61. Based on this observation a mouse model for NSCLC was recently established (Sweet-Cordero *et al.,* 2004). The local activation of a silent transgenic *K-rasG12D* allele in these mice is achieved by intratracheal adenoviral transfection with the Cre recombinase (AdCre). The Cre recombinase cuts out a "stop" cassette and allows for expression of the oncogenic K-ras protein in transfected lung cells. When the initiation is performed at the age of 8 weeks, mice are diagnosed 16 weeks later with lung tumors at 100 % penetrance. The morphology and molecular characteristics of the tumors are very similar to human NSCLC and they respond with regression to chemotherapy with cis-platin. Because cis-platin is a first line drug in treating human NSCLC, this mouse model was employed to investigate possible effects of DPH on the treatment outcome of chemotherapy with cis-platin.

### No effect of DPH on the DNA platination in primary lung tumor cells

Mice bearing *K-rasG12D* -induced lung tumors were divided into two groups for acute high dose and for chronic low dose treatment. Animals received mocktreatment with PBS or cis-platin alone or cis-platin plus treatment with DPH. For acute treatment, the mice were injected i.p. once with cis-platin (10 mg/kg) and for chronic treatment every second day with 5 mg/kg (4 consecutive injections, cumulative dose: 20 mg/kg). DPH was given at a dose of 57 mg/kg 30 minutes prior to each dose of cis-platin. All mice were sacrificed 24 hours after the last treatment and cochleae, kidneys and lungs were resected for analysis of DNA adduct accumulation. Whereas the protective effect of DPH on the DNA platination in renal and cochlear target cells was present as expected from the previous experiments, no reduction of the Pt-GG levels by DPH co-treatment was measurable in lung tumor cells, independent from the treatment schedule (Figure 13 A). Adduct levels were about two-fold higher after repetitive treatment as compared to single high dose treatment. These observations suggest that the pharmacodynamics of cis-platin in primary lung tumor cells of mice is different from the situation in the kidney and the cochlea, allowing for the severe side effects of the drug. Active uptake of the drug by a DPH sensitive transporter does not seem to play a major role in the malignant lung cells, but could be confirmed for kidney and cochlear cells.

### Effect of DPH on cis-platin controlled tumor growth

In addition to the analysis of adduct levels in the *K-ras* mouse model, it was analyzed whether the co-administration of DPH negatively affected the therapeutic efficacy of cis-platin in the NSCLC tumors. A cohort of tumor-bearing mice was randomly divided into three subgroups receiving different treatment regimens. Group one (mock treatment control) received i.p. injections once per week with PBS for 4 weeks. The second group received cis-platin treatment (7 mg/kg) once per week for 4 weeks, and mice of the third group were additionally pre-treated with DPH (57 mg/kg) 30 minutes before each dose of cis-platin. Mice were sacrificed 5 days after the last dose; lungs were dissected and prepared for paraffin sections. Following H&E staining for morphological structures, the percentage tumor burden, the number of tumors per lung and the average tumor size was determined microscopically (Figure 13 B-D, 14). In addition, kidneys were resected from all mice as internal control for correct cis-platin treatment and cryosections were analyzed by ICA for DNA platination. By microscopic inspection of the stained lung section, it became obvious that the number of tumors per lung were higher in PBS-treated mice as compared to animals of the two cis-platin treated groups (Figure 14). For quantitative analysis the Bioquant image analysis software was used to blindly and randomly quantify the area of all lung tumors and the total lung surface area in 4 lung sections per mouse. The measurement revealed significantly lower average tumor sizes in both groups with cis-platin treatment as compared to the PBS control group and no difference between the cis-platin alone and the cis-platin plus inhibitor group (Figure 13 B-D). The mean tumor burden in the lung, (as calculated from total area of tumor divided by total lung area), too, was significantly lower in both groups of cis-platin treated mice with no significant difference if given alone or in combination with DPH (Figure 13B-D). Tumor number was reduced in cis-platin and cis-platin plus DPH treated mice, but these values were not statistically significant. This suggests that the drugs mainly reduce tumor growth and have less of an effect on tumor initiation. In summary, the co-application of DPH neither influenced the DNA damage level induced by cis-platin in primary lung tumors nor did it significantly hamper the therapeutic efficacy of the drug as determined by tumor burden and average lung tumor size. These results show that during cancer chemotherapy with cis-platin the concomitant application of DPH is a suitable and safe pharmacological strategy to prevent structural and functional damage in the inner ear and in the kidney without affecting the therapeutic response.

### 2.7 Effect of DPH on survival upon cis-platin treatment

Repetitive high doses of cis-platin sufficiently reduce tumor growth and burden. However, despite this, still high death rates are observed upon cis-platin treatment, probably partially due to the cell damaging effects of the drug itself. Therefore, it is particularly interesting that the inventor observed improved survival rates of animals treated with cis-platin in combination with DPH (Figure 16). Thus, DPH is suitable to antagonize the oto-, nephro- and neurotoxic side effects of cis-platin and, in addition, to improve survival prognosis.

### 3. Summary and therapeutic application

### Cell type-specific accumulation of Pt-DNA adducts

The inventor showed that the formation rate of cis-platin induced DNA damage is cell-type specific, and predominantly occurs in two types of the inner ear, namely the marginal cells of the stria vascularis and the outer hair cells of the organ of Corti. To further clarify the molecular mechanisms of cis-platin ototoxicity, a mouse model was established, which allowed the analysis of electrophysiological parameters of hearing capability and, simultaneously, of the formation and processing of Pt-DNA adducts at the level of individual cells. The degree of cis-platin induced DNA damage and its repair was measured in different types of inner ear and kidney cells. This study shows for the first time that the DNA in outer hair cells and marginal cells of the cochlea is extensively damaged in cis-platin treated mice. Up to eight-fold differences between the various cells layers in the stria vascularis as well as between the outer hair cells and other cell types of the organ of Corti were observed. A similar distribution of the DNA platination was detected throughout kidney cortex cells, with high levels in proximal tubular cells as compared to other kidney cortex cells. These observations strictly contradict the assumption of passive diffusion as the exclusive or main route of cis-platin to enter such cells. More likely, the active import of cis-platin by membrane transporters may play a role for the distinct distribution of DNA platination products within a given tissue. The finding of similarly high platinated cells within two structurally and functionally unrelated tissues indicates a causative role of channels or transporters expressed in the kidney as well as in the cochlea. A number of clinical observations documented that almost all ototoxic agents also have nephrotoxic effects. Due to its comparatively small size (MW: 300), the inventor believes that cis-platin is aberrantly recognized as a substrate by specific membrane transporters in the target cells of the inner ear and the kidney.

### The role of the NER protein XPA in the cells of the hearing system

Functional DNA repair is critical for the viability of cells, particularly during acute genotoxic stress. The XPA protein plays a central role in the NER pathway by recruiting several other NER proteins, which build the repair incision complex at the damaged site. XPA-knockout mice defective in both sub-pathways of the NER, were used to study the influence of this repair system on the cytotoxicity of cis-platin and the processing of Pt-GG adducts. The experiments revealed that XPA-deficient mice were highly sensitive to the general toxicity of cis-platin, accompanied by a high accumulation of adducts in cochlear cells and a decrease in hearing performance. The inventor shows strong support for the hypothesis that the cis-platin induced DNA adducts initially trigger cellular processes finally leading to functional sensorineural hearing loss.

### Screening for a pharmacological inhibitor of cis-platin induced ototoxicity

Several compounds were examined in an established mouse model for cis-platin induced ototoxicity, showing for the first time that the co-application of diphenhydramine (DPH) significantly reduces both, the excessive DNA platination in the critical inner ear cells and the functional loss in the hearing system. In addition, it was shown that DPH had no effect on the DNA repair capacity of cochlear and kidney cells. However, the cells in focus, namely the outer hair cells and marginal cells of the inner ear and the proximal tubular cells of the kidney, are all characterized by their distinct profiles of multiple membrane transporters ensuring their functionality in the respective tissues. Therefore, and supported by the strictly dose-dependent pharmacological inhibition of DNA platination by DPH in the target cells, without exerting any influence on other surrounding cells, the inventor presumes without being bound to any theory that DPH binds to, and thereby blocks, one or more specific transporters on the target cells.

### DPH prevents cis-platin induced ototoxicity in mice

Cis-platin chemotherapy is usually applied in the clinic in repetitive treatment cycles, and thus, leads to progressive and permanent hearing loss. In cases of successful therapy, most of the patients, and in particular treated children, suffer from the toxic side effects for the rest of their lives. In this study, clinically relevant degrees of hearing loss were measurable in mice treated repetitively with low doses of cis-platin and a strong reduction of this side effect was observed when DPH was given prior to each treatment cycle. Furthermore, it was demonstrated for the first time that unrepaired platinum-DNA adducts in the target cells represent the critical triggers for cis-platin toxicity and that the severity of the side effects is strictly correlated to the amount of drug-induced DNA damage. Micromorphological analyses by electron microscopy revealed numerous large autophagosomes containing a bulk of cytoplasmic organelles in these cells (Figure 15). Such alterations are generally indicative for autophagic or necrotic processes rather than for survival or apoptosis. No signs of autophagy or necrosis were recorded in the respective cells from animals treated with DPH alone or from untreated controls, and autophagosomes were much less frequent and properly shaped in cochlear cells of mice treated with cis-platin plus DPH.

### DPH does not impair the anti-neoplastic effect of cis-platin

Since the severe side effects of cis-platin were already recognized in early clinical trials, numerous pharmacological, molecular, and genetic approaches have been-suggested to circumvent the neuro-, nephro- and ototoxicity of the drug. None of these preventive strategies, however, reached the clinical level, although the majority of them had demonstrated their effectiveness in animal models. The major obstacles derived from warrantable concerns regarding putative negative effects of such strategies on the tumor-therapeutic efficacy of cis-platin, Therefore, there was an urgent need to address this problem carefully when developing novel approaches. Lung cancer is the leading cause of cancer death world wide, and non-small cell lung cancer (NSCLC) accounts for approximately 80 % of the cases). *This* malignancy is frequently associated with mutations in the gene coding for the small GTPase *K-ras* and is usually treated with cis-platin-based combination therapies. The use of genetically engineered mice carrying a silent oncogenic *K-ras* transgene, which can be activated in a controlled manner, provides a model that closely recapitulates many aspects of human lung oncogenesis. The molecular similarity between *K-ras*-initiated murine lung tumors and the human NSCLC suggests that this is a relevant model to investigate the molecular mechanisms of chemotherapy response. In order to explore whether DPH might have an effect on the anti-cancer activity of cis-platin in this model, renal, cochlear and lung tumor tissues were examined for DNA adduct accumulation after short-term and long-term treatments. Furthermore, the average tumor volume and the percentage of tumor burden were measured in animals treated with cis-platin alone or in combination with the inhibitor. Moreover, DPH did not influence the tumor response during several cycles of chemotherapy with cis-platin. In conclusion, this study revealed a novel highly protective role of DPH for the target cells of cis-platin toxicity, while retaining the pro-apoptotic role of cis-platin in primary lung tumor cells *in vivo*.

### References

Arany, I. and Safirstein, R.L. Cisplatin nephrotoxicity. Semin Nephrol 23, 460-464 (2003).
Cardinaal, R.M., et al. Ultrastructural changes in the albino guinea pig cochlea at different survival times following cessation of 8-day cisplatin administration. Acta Otolaryngol 124, 144-154 (2004).
Dzagnidze, A., et al. Repair capacity for platinum-DNA adducts determines the severity of cisplatin-induced peripheral neuropathy. J Neurosci 27, 9451-9457 (2007).
Jentsch TJ., Neuronal KCNQ potassium channels: physiology and role in disease. Nat Rev Neurosci. 2000 Oct;1(1):21-30
Kalinec, GM., et al. A cochlear cell line as an in vitro system for drug ototoxicity screening. Audiol Neurootol. 8(4),177-189 (2003).
Kim, H.J., et al. Roles of NADPH oxidases in cisplatin-induced reactive oxygen species generation and ototoxicity. J Neurosci 30, 3933-3946 (2010).
Klis, S.F., et al. Partial recovery of cisplatin-induced hearing loss in the albino guinea pig in relation to cisplatin dose. Hear Res 164, 138-146 (2002).
Liedert, B., et al. Adduct-specific monoclonal antibodies for the measurement of cisplatin-induced DNA lesions in individual cell nuclei. Nucleic Acids Res 34, 47 (2006).
Oliver, T.G., et al. Chronic cisplatin treatment promotes enhanced damage repair and tumor progression in a mouse model of lung cancer. Genes Dev 24, 837-852 (2010).
Sluyter, S., et al. Alterations in the stria vascularis in relation to cisplatin ototoxicity and recovery. Hear Res 185, 49-56 (2003).
Sweet-Cordero, A., et al. An oncogenic KRAS2 expression signature identified by cross-species gene-expression analysis. Nat Genet 37, 48-58 (2004).
Thomas, J.P., et al. High accumulation of platinum-DNA adducts in strial marginal cells of the cochlea is an early event in cisplatin but not carboplatin ototoxicity. Mol Pharmacol 70, 23-29 (2006).

## Claims

1. Diphenylhydramine (DPH) for treating ototoxicity in a subject, wherein the ototoxicity is induced by a medication.

2. DPH according to claim 1, wherein the medication comprises an anti-cancer agent, preferably a platinum complex, a nitrogen mustard derivative or vincristine; an antibiotic, an ototoxic diuretic and/or a quinine derivative.

3. DPH according to claim 2, wherein the platinum complex is selected from the group consisting of cis-diaminedichloro-platinum(II) (cis-platin), cis-platin analogs, amine platinum complexes, diamine platinum complexes, trans-platinum complexes multinuclear platinum complexes and Platinum(IV) coordination complexes.

4. DPH according to any of claims 1 to 3, wherein the medication is administered during a cancer treatment.

5. DPH according to claim 1, wherein DPH is administered to the subject before or concurrently with the medication.

6. DPH according to claim 1 for additionally treating nephrotoxicity.

7. DPH for inhibiting the formation of platinum adducts in a cell of the inner ear.

8. DPH for preventing and/or inhibiting structural and functional damage in the inner ear during a platinum-based cancer treatment.

9. An agent for treating ototoxicity induced by a platinum complex, wherein the agent interferes with a modulator of the import and/or the export of the platinum complex into/from a cell.

10. DPH for treating nephrotoxicity in a subject induced by a medication comprising a platinum complex, wherein DPH inhibits the formation of platinum adducts within a cell of the kidney, preferably of the kidney cortex, more preferred a proximal tubular cells.

11. DPH for treating ototoxicity and/or nephrotoxicity in a subject, wherein the ototoxicity and/or nephrotoxicity is induced by a medication comprising a platinum complex.

12. An *in vitro* method for determining a subject's susceptibility for platinum induced ototoxicity and/or platinum induced nephrotoxicity, comprising the steps of
- determining the subject's genetic variant of a platinum complex transporter, and
- correlating the genetic variant of the platinum complex transporter to the susceptibility for platinum induced ototoxicity and/or platinum induced nephrotoxicity.

13. An *in vitro* method for screening a compound capable of inhibiting ototoxicity, nephrotoxicity and/or neurotoxicity induced by a platinum complex, comprising the steps of
- providing a test sample comprising cells derived from the organ of Corti, the stria vascularis of the inner ear and/or the proximal tubules of the kidney,
- incubating the test sample with the compound,
- adding the platinum complex to the test sample,
- determining the amount of platinum adducts formed by the platinum complex in the test sample, and
- comparing the level of platinum adducts formed in the test sample to the level of platinum adducts formed in a control sample,
wherein a reduced amount of platinum adducts formed in the test sample in comparison to the control sample indicates the compound's potential to inhibit ototoxicity induced by a platinum complex.

14. A kit for screening a compound, comprising
- cells derived from the organ of Corti, the stria vascularis of the inner ear and/or the proximal tubules of the kidney,
- a platinum complex, and
- an antibody against platinum adducts.

15. A composition for treating cancer and concurrently inhibiting ototoxicity, nephrotoxicity and/or neurotoxicity, wherein the composition comprises cis-platin and DPH.
